(19) Europäisches Patentamt
European Patent Office
Office européen des brevets

(11) EP 1 724 270 A2

(12) # EUROPEAN PATENT APPLICATION

(43) Date of publication:
22.11.2006 Bulletin 2006/47

(51) Int Cl.:
*C07D 417/12* (2006.01) *C07D 277/46* (2006.01)

(21) Application number: 06120487.1

(22) Date of filing: 02.07.2002

(84) Designated Contracting States:
AT BE BG CH CY CZ DE DK EE ES FI FR GB GR IE IT LI LU MC NL PT SE SK TR
Designated Extension States:
AL LT LV MK RO SI

(30) Priority: 19.07.2001 US 907947
20.02.2002 US 357642 P

(62) Document number(s) of the earlier application(s) in accordance with Art. 76 EPC:
02760213.5 / 1 406 899

(71) Applicant: **Pfizer Italia S.r.l.**
**04010 Latina (IT)**

(72) Inventors:
- **Pevarello, Paolo**
  **27100 Pavia (IT)**
- **Amici, Raffaella**
  **20073 Codogno (Lodi) (IT)**
- **Villa, Manuela**
  **22040 Lurago d'Erba (Como) (IT)**
- **Salom, Barbara**
  **20057 Vedano al Lambro (Milano) (IT)**
- **Vulpetti, Anna**
  **20047 Brugherio (Milano) (IT)**
- **Varasi, Mario**
  **20142 Milano (IT)**
- **Brasca, Maria, Gabriella**
  **20090 Cusago (Milano) (IT)**
- **Traquandi, Gabriella**
  **20152 Milano (IT)**
- **Nesi, Marcella**
  **21047 Saronno (Varese) (IT)**

(74) Representative: **Modiano, Micaela Nadia**
**Modiano Josif Pisanty & Staub Ltd**
**Thierschstrasse 11**
**80538 München (DE)**

Remarks:
This application was filed on 12 - 09 - 2006 as a divisional application to the application mentioned under INID code 62.

(54) **Phenylacetamido-thiazole derivatives, process for their preparation and their use as antitumor agents**

(57) Compounds represented by formula (I), as defined in the description, wherein R is a hydrogen atom or a methyl group and R1 is a group as defined in the specification, or a pharmaceutically acceptable salt thereof, are disclosed; the said compounds are useful in the treatment of cell proliferative disorders, e.g. cancer, associated with an altered cell cycle dependent kinase activity.

EP 1 724 270 A2

**Description**

BACKGROUND OF THE INVENTION

Field of the Invention

The present invention relates to phenylacetamido-thiazole derivatives, to a process for their preparation, to pharmaceutical compositions containing them, and to their use as therapeutic agents, particularly in the treatment of cancer and cell proliferation disorders.

Discussion of the Background

Several cytotoxic drugs such as, e.g., fluorouracil (5-FU), doxorubicin and camptothecins, damage DNA or affect cellular metabolic pathways and thus cause, in many cases, an indirect block of the cell cycle. Therefore, by producing an irreversible damage to both normal and tumor cells, these agents result in a significant toxicity and side-effects.
In this respect, compounds capable of functioning as highly specific antitumor agents by selectively leading to tumor cell arrest and apoptosis, with comparable efficacy but reduced toxicity than the currently available drugs, are desirable.
It is well known that progression through the cell cycle is governed by a series of checkpoint controls, otherwise referred to as restriction points, which are regulated by a family of enzymes known as the cyclin-dependent kinases (cdk). In turn, the cdks themselves are regulated at many levels such as, for instance, binding to cyclins.
The coordinated activation and inactivation of different cyclin/cdk complexes is necessary for normal progression through the cell cycle. Both the critical G1-S and G2-M transitions are controlled by the activation of different cyclin/cdk activities. In G1, both CdK4/cyclin D and CdK2/cyclin E are thought to mediate the onset of S-phase. Progression through S-phase requires the activity of CdK2/cyclin A whereas the activation of cdc2 (cdkl)/cyclin A and cdc2/cyclin B are required for the onset of mitosis. For a general reference to cyclins and cyclin-dependent kinases see, for instance, Kevin R. Webster et al, in Exp. Opin. Invest. Drugs, 1998, Vol. 7(6), 865-887.
Checkpoint controls are defective in tumor cells due, in part, to disregulation of cdk activity. For example, altered expression of cyclin E and cdks has been observed in tumor cells, and deletion of the cdk inhibitor p27 KIP gene in mice has been shown to result in a higher incidence of cancer.
Increasing evidence supports the idea that the cdks are rate-limiting enzymes in cell cycle progression and, as such, represent molecular targets for therapeutic intervention. In particular, the direct inhibition of cdk/cyclin kinase activity should be helpful in restricting the unregulated proliferation of a tumor cell.

SUMMARY OF THE INVENTION

It is an object of the invention to provide compounds which are useful in treating cell proliferative disorders associated with an altered cell cycle dependent kinase activity. It is another object to provide compounds which have cdk/cyclin kinase inhibitory activity. The present inventors have now discovered that certain phenylacetamido-thiazoles are endowed with cdk/cyclin kinase inhibitory activity and are thus useful in therapy as antitumor agents and lack, in terms of both toxicity and side effects, the aforementioned drawbacks associated with currently available antitumor drugs.
More specifically, the phenylacetamido-miazoles of the invention are useful in the treatment of a variety of cancers including, but not limited to: carcinoma such as bladder, breast, colon, kidney, liver, lung, including small cell lung cancer, esophagus, gall-bladder, ovary, pancreas, stomach, cervix, thyroid, prostate, and skin, including squamous cell carcinoma; hematopoietic tumors of lymphoid lineage including leukaemia, acute lymphocitic leukaemia, acute lymphoblastic leukaemia, B-cell lymphoma, T-cell-lymphoma, Hodgkin's lymphoma, non-Hodgkin's lymphoma, hairy cell lymphoma and Burkett's lymphoma; hematopoietic tumors of myeloid lineage, including acute and chronic myelogenous leukemias, myelodysplastic syndrome and promyelocytic leukaemia; tumors of mesenchymal origin, including fibrosarcoma and rhabdomyosarcoma; tumors of the central and peripheral nervous system, including astrocytoma neuroblastoma, glioma and schwannomas; other tumors, including melanoma, seminoma, teratocarcinoma, osteosarcoma, xeroderma pigmentosum, keratocanthoma, thyroid follicular cancer and Kaposi's sarcoma.
Due to the key role of cdks in the regulation of cellular proliferation, these phenylacetamido-thiazole derivatives are also useful in the treatment of a variety of cell proliferative disorders such as, for example, benign prostate hyperplasia, familial adenomatosis polyposis, neurofibromatosis, psoriasis, vascular smooth cell proliferation associated with atherosclerosis, pulmonary fibrosis, arthritis, glomerulonephritis and post-surgical stenosis and restenosis.
The compounds of the invention may be useful in treatment of Alzheimer's disease, as suggested by the fact that cdk5 is involved in the phosphorylation of tau protein (J. Biochem. 117, 741-749, 1995).
The compounds of this invention, as modulators of apoptosis, may also be useful in the treatment of cancer, viral

infections, prevention of AIDS development in HIV-infected individuals, autoimmune diseases and neurodegenerative disorders.
The compounds of this invention may be useful in inhibiting tumor angiogenesis and metastasis.
The compounds of the invention may also act as inhibitor of other protein kinases, e.g., protein kinase C in different isoforms, Met, PAK-4, PAK-5, ZC-1, STLK-2, DDR-2, Aurora 1, Aurora 2, Bub-1, PLK, Chk1, Chk2, HER2, raf1, MEK1, MAPK, EGF-R, PDGF-R, FGF-R, IGF-R, PI3K, weel kinase, Src, Abl, Akt, MAPK, ILK, MK-2, IKK-2, Cdc7, Nek, and thus be effective in the treatment of diseases associated with other protein kinases.
The compounds of the invention are also useful in the treatment and prevention of radiotherapy-induced or chemotherapy-induced alopecia.

Accordingly, the present invention provides a method for treating cell proliferative disorders associated with an altered cell cycle dependent kinase activity, by administering to a mammal in need thereof an effective amount of a phenylacetamido-thiazole derivative represented by formula (I)

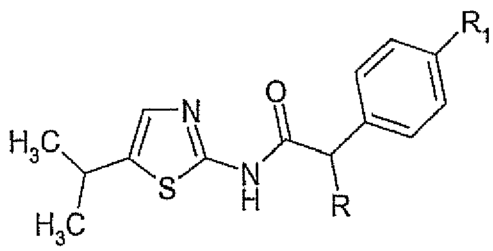

(I)

wherein
R is a hydrogen atom or a straight or branched $C_1$-$C_4$ alkyl group;
$R_1$ is a group of formula (IIa-e)

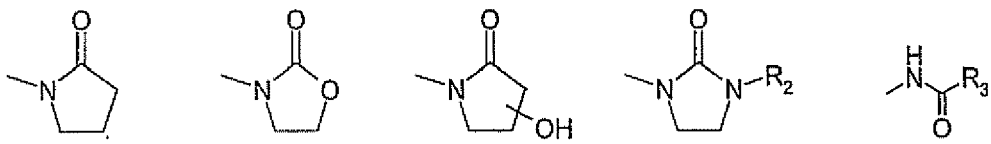

(IIa) (IIb) (IIc) (IId) (IIe)

wherein $R_2$ is hydrogen or a straight or branched $C_1$-$C_6$ alkyl group and the hydroxy group onto ring (IIc) is in any one of the free positions;
$R_3$ is selected from the group consisting of amino, aminomethyl (-$CH_2$-$NH_2$), hydroxymethyl (-$CH_2OH$), straight or branched $C_1$-$C_4$ alkyl or it is a 5 or 6 membered heterocycle with 1 or 2 heteroatoms selected among nitrogen, oxygen and sulfur;
provided that when R is hydrogen, then $R_3$ is other than methyl or pyridyl-3-yl; or a pharmaceutically acceptable salt thereof.

In a preferred embodiment of the method described above, the cell proliferative disorder is selected from the group consisting of cancer, Alzheimer's disease, viral infections, auto-immune diseases and neurodegenerative disorders.
Specific types of cancer that may be treated include carcinoma, squamous cell carcinoma, hematopoietic tumors of myeloid or lymphoid lineage, tumors of mesenchymal origin, tumors of the central and peripheral nervous system, melanoma, seminoma, teratocarcinoma, osteosarcoma, xeroderma pigmentosum, keratocanthoma, thyroid follicular cancer, and Kaposi's sarcoma.
In another preferred embodiment of the method described above, the cell proliferative disorder is selected from the group consisting of benign prostate hyperplasia, familial adenomatosis polyposis, neuro-fibromatosis, psoriasis, vascular smooth cell proliferation associated with atherosclerosis, pulmonary fibrosis, arthritis, glomerulonephritis and post-surgical stenosis and restenosis.
In addition, the inventive method provides tumor angiogenesis and metastasis inhibition. The inventive method may also provide cell cycle inhibition or cdk/cyclin dependent inhibition.
In addition to the above, the method object of the present invention provides treatment and prevention of radiotherapy-induced or chemotherapy-induced alopecia.

The present invention also provides a phenylacetamido-thiazole derivative represented by formula (I)

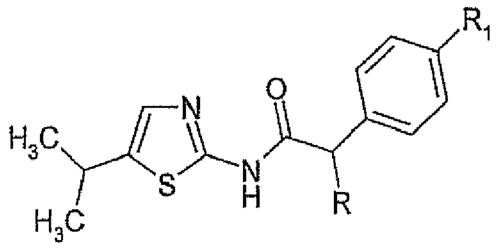

(I)

wherein
R is a hydrogen atom or a methyl group;
$R_1$ is a group of formula (IIa-e)

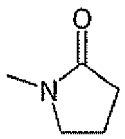
(IIa)

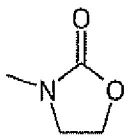
(IIb)

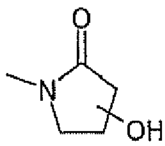
(IIc)

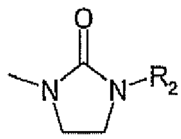
(IId)

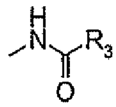
(IIe)

wherein $R_2$ is hydrogen or a straight or branched $C_1$-$C_6$ alkyl group and the hydroxy group onto ring (IIc) is in any one of the free positions;
$R_3$ is selected from the group consisting of amino, aminomethyl ($-CH_2-NH_2$), hydroxymethyl ($-CH_2OH$), straight or branched $C_1$-$C_4$ alkyl or it is a 5 or 6 membered heterocycle with 1 or 2 heteroatoms selected among nitrogen, oxygen and sulfur;
provided that when R is hydrogen, then $R_3$ is other than methyl or pyridyl-3-yl; or a pharmaceutically acceptable salt thereof.

The present invention also includes methods of synthesising the phenylacetamido-thiazole derivatives represented by formula (I). A pharmaceutical composition comprising the phenylacetamido-thiazole derivatives represented by formula (I) is also included in the present invention.

A more complete appreciation of the invention and many of the attendant advantages thereof will be readily obtained as the same becomes better understood by reference to the following detailed description.

## DETAILED DESCRIPTION OF THE INVENTION

Several aminothiazoles are known in the art, for instance as herbicides, synthetic intermediates or even as therapeutic agents. Among these are, as an example, 2-benzamido-1,3-thiazoles as antiallergic agents (EP-A-261503, Valeas S.p.A.); 5-alkyl-2-phenylalkylcarbonylamino-1,3-thiazoles as protein kinase C inhibitors (WO 98/04536, Otsuka Pharmaceutical Co.); 5-arylthio-2-acylamino-1,3-thiazoles as antitumor agents (EP-A-412404, Fujisawa Pharm. Co.); 4-amino-2-carbonylamino-1,3-thiazoles as cyclin-dependent kinase inhibitors (WO 99/21845, Agouron Pharmaceutical Inc.); aminothiazoles among which are 5-alkenyl-2-acylamino-thiazoles as cyclin dependent kinases inhibitors (WO 99/65884, Bristol-Myers Squibb Co.); phenylacetamido-thiazoles substituted by cycloalkyl-alkyl groups as glucokinase activators, useful in the treatment of type II diabetes (WO 01/85707, Hoffmann-La Roche AG).
In addition, two international patent applications WO 00/26202 and WO 01/14353 (US 6,114,365), both in the name of Pharmacia & Upjohn S.p.A. and herewith incorporated by reference, disclose broad classes of amido-thiazole derivatives possessing cell dependent kinase inhibitory activity.
The compounds object of the present invention fall within the scope of the general formula of WO 00/26202 and WO 01/14353 but are not specifically exemplified therein.

The compounds of formula (I) may have asymmetric carbon atoms and may therefore exist either as racemic admixtures or as individual optical isomers which are all within the scope of the present invention.

As an example, when R is other than hydrogen, the carbon atom to which R itself is attached is an asymmetric carbon atom and, hence, both the (R) and (S) optical isomers of the compounds of formula (I), as well as the racemic

(R,S) admixture or any other admixture comprising a majority of one of the two optical (R) or (S) isomers, are within the scope of the invention.

Likewise, the use as an antitumor agent of all the possible isomers and their admixtures and of both the metabolites and the pharmaceutically acceptable bio-precursors (otherwise referred to as pro-drugs) of the compounds of formula (I) are also within the scope of the present invention.

In the present description, as it is clear to the skilled man, the nitrogen atom being part of the $R_1$ moieties of formula (IIa), (IIb), (IIc), (IId) and (IIe) is directly bonded, in formula (I), to the phenylene moiety.

In addition, unless otherwise specified, when referring to the compounds of formula (I) wherein $R_1$ is a group (IIc), the 2-oxo-pyrrolidin-1-yl moiety is further substituted in position 3, 4 or 5 of the ring with hydroxy groups so as to get a 3-hydroxy-, 4-hydroxy- or 5-hydroxy-2-oxo-pyrrolidin-1-yl group.

In the present description, unless otherwise specified, with the term straight or branched $C_1$-$C_4$ alkyl we intend any of the groups methyl, ethyl, n-propyl, isopropyl, n-butyl, isobutyl, tert-butyl and sec-butyl.

With the term 5 or 6 membered heterocycle with 1 or 2 heteroatoms selected among nitrogen, oxygen or sulfur, we intend a group such as, for instance, furan, pyrrole, imidazole, pyrazole, thiophene, thiazole, isothiazole, oxazole, isoxazole, pyridine, pyrazine, pyrimidine, pyridazine, pyrrolidine, pyrroline, imidazolidine, imidazoline, pyrazolidine, pyrazoline, piperidine, piperazine, morpholine, and the like.

Pharmaceutically acceptable salts of the compounds of formula (I) include the acid and basic addition salts with inorganic or organic acids or bases, respectively. Examples of the above acids include, for instance, nitric, hydrochloric, hydrobromic, sulphuric, perchloric, phosphoric, acetic, trifluoroacetic, propionic, glycolic, lactic, oxalic, malonic, malic, maleic, tartaric, citric, benzoic, cinnamic, mandelic, methanesulphonic, isethionic and salicylic acid.

Likewise, suitable bases are, for instance, alkaline or alkaline-earth metals hydroxides as well as organic amines, e.g. aliphatic amines, piperidine, and the like.

A class of preferred compounds of formula (I) of the invention are those wherein R is methyl.

Within this class of preferred compounds of formula (I), most preferred are those wherein $R_1$ is a group of formula (IIa), (IIb) or (IIc) wherein the hydroxy substituent is in position 3 of the pyrrolidine ring, or it is a group of formula (IId) wherein $R_2$ is hydrogen or methyl, or it is a group of formula (IIe) wherein $R_3$ is methyl or pyridyl, hence including pyridyl-4-yl, pyridyl-3-yl or pyridyl-2-yl.

Still more preferred, within this class, are the compounds of formula (I) wherein $R_1$ is a group of formula (IIa) or (IIe).

Also preferred are the compounds of formula (I) wherein R is a hydrogen atom.

Still more preferred are the compounds wherein the above features are combined together so as to get a compound of formula (I) wherein R is hydrogen and $R_1$ is a group of formula (IIa), that is the compound N-(5-isopropyl-1,3-thiazol-2-yl)-2-[4-(2-oxo-1-pyrrolidinyl)phenyl]acetamide, or a compound of formula (I) wherein R is methyl and $R_3$ is methyl, pyridyl-3-yl or pyridyl-4-yl, in their (S) configuration, namely (2S)-2-[4-(acetylamino)phenyl]-N-(5-isopropyl-1,3-thiazol-2-yl)propanamide, N-(4-{(1S)-2-[(5-isopropyl-1,3-thiazol-2-yl)amino]-1-methyl-2-oxoethyl}phenyl)nicotinamide and N-(4-{(1S)-2-[(5-isopropyl-1,3-thiazol-2-yl)amino]-1-methyl-2-oxoethyl}phenyl)isonicotinamide.

Examples of compounds of formula (I) of the invention, optionally in the form of pharmaceutically acceptable salts, are:

1. N-(5-isopropyl-1,3-thiazol-2-yl)-2-[4-(2-oxo-1-pyrrolidinyl)phenyl]acetamide;
2. N-(5-isopropyl-1,3-thiazol-2-yl)-2-[4-(2-oxo-1-pyrrolidinyl)phenyl]propanamide;
3. (2R)-N-(5-isopropyl-1,3-thiazol-2-yl)-2-[4-(2-oxo-1-pyrrolidinyl)phenyl]propanamide;
4. (2S)-N-(5-isopropyl-1,3-thiazol-2-yl)-2-[4-(2-oxo-1-pyrrolidinyl)phenyl]propanamide;
5. 2-[4-(3-hydroxy-2-oxo-1-pyrrolidinyl)phenyl]-N-(5-isopropyl-1,3-thiazol-2-yl)acetamide;
6. 2-{4-[(3S)-3-hydroxy-2-oxo-1-pyrrolidinyl]phenyl}-N-(5-isopropyl-1,3-thiazol-2-yl)acetamide;
7. 2-{4-[(3R)-3-hydroxy-2-oxo-1-pyrrolidinyl]phenyl}-N-(5-isopropyl-1,3-thiazol-2-yl)acetamide;
8. 2-[4-(3-hydroxy-2-oxo-1-pyrrolidinyl)phenyl]-N-(5-isopropyl-1,3-thiazol-2-yl)propanamide;
9. 2-{4-[(3S)-3-hydroxy-2-oxo-1-pyrrolidinyl]phenyl}-N-(5-isopropyl-1,3-thiazol-2-yl)propanamide;
10. 2-{4-[(3R)-hydroxy-2-oxo-1-pyrrolidinyl]phenyl}-N-(5-isopropyl-1,3-thiazol-2-yl)propanamide;
11. (2R)-2-{4-[(3S)-3-hydroxy-2-oxo-1-pyrrolidinyl]phenyl}-N-(5-isopropyl-1,3-thiazol-2-yl)propanamide;
12. (2S)-2-{4-[(3S)-3-hydroxy-2-oxo-1-pyrrolidinyl]phenyl}-N-(5-isopropyl-1,3-thiazol-2-yl)propanamide;
13. (2R)-2-{4-[(3R)-3-hydroxy-2-oxo-1-pyrrolidinyl]phenyl}-N-(5-isopropyl-1,3-thiazol-2-yl)propanamide;
14. (2S)-2-{4-[(3R)-3-hydroxy-2-oxo-1-pyrrolidinyl]phenyl}-N-(5-isopropyl-1,3-thiazol-2-yl)propanamide;
15. N-(5-isopropyl-1,3-thiazol-2-yl)-2-[4-(2-oxo-1,3-oxazolidin-3-yl)phenyl]acetamide;
16. N-(5-isopropyl-1,3-thiazol-2-yl)-2-[4-(2-oxo-1,3-oxazolidin-3-yl)phenyl]propanamide;
17. (2R)-N-(5-isopropyl-1,3-thiazol-2-yl)-2-[4-(2-oxo-1,3-oxazolidin-3-yl)phenyl]propanamide;
18. (2S)-N-(5-isopropyl-1,3-thiazol-2-yl)-2-[4-(2-oxo-1,3-oxazolidin-3-yl)phenyl]propanamide;
19. N-(5-isopropyl-1,3-thiazol-2-yl)-2-[4-(2-oxo-1-imidazolidinyl)phenyl]acetamide;

20. N-(5-isopropyl-1,3-thiazol-2-yl)-2-[4-(2-oxo-1-imidazolidinyl)phenyl]propanamide;
21. (2R)-N-(5-isopropyl-1,3-thiazol-2-yl)-2-[4-(2-oxo-1-imidazolidinyl)phenyl]propanamide;
22. (2S)-N-(5-isopropyl-1,3-thiazol-2-yl)-2-[4-(2-oxo-1-imidazolidinyl)phenyl]propanamide;
23. N-(5-isopropyl-1,3-thiazol-2-yl)-2-[4-(3-methyl-2-oxo-1-imidazolidinyl)phenyl]acetamide;
24. N-(5-isopropyl-1,3-thiazol-2-yl)-2-[4-(3-methyl-2-oxo-1-imidazolidinyl)phenyl]propanamide;
25. (2R)-N-(5-isopropyl-1,3-thiazol-2-yl)-2-[4-(3-methyl-2-oxo-1-imidazolidinyl)phenyl]propanamide;
26. (2S)-N-(5-isopropyl-1,3-thiazol-2-yl)-2-[4-(3-methyl-2-oxo-1-imidazolidinyl)phenyl]propanamide;
27. (2S)-2-[4-(acetylamino)phenyl]-N-(5-isopropyl-1,3-thiazol-2-yl)propanamide;
28. (2R)-2-[4-(acetylamino)phenyl]-N-(5-isopropyl-1,3-thiazol-2-yl)propanamide;
29. 2-[4-(acetylamino)phenyl]-N-(5-isopropyl-1,3-thiazol-2-yl)propanamide;
30. (2S)-2-{4-[(aminocarbonyl)amino]phenyl}-N-(5-isopropyl-1,3-thiazol-2-yl)propanamide;
31. (2R)-2-{4-[(aminocarbonyl)amino]phenyl}-N-(5-isopropyl-1,3-thiazol-2-yl)propanamide;
32. 2-{4-[(aminocarbonyl)amino]phenyl}-N-(5-isopropyl-1,3-thiazol-2-yl)propanamide;
33. 2-{4-[(aminocarbonyl)amino]phenyl}-N-(5-isopropyl-1,3-thiazol-2-yl)acetamide;
34. (2S)-2-[4-(glycylamino)phenyl]-N-(5-isopropyl-1,3-thiazol-2-yl)propanamide;
35. (2R)-2-[4-(glycylamino)phenyl]-N-(5-isopropyl-1,3-thiazol-2-yl)propanamide;
36. 2-[4-(glycylamino)phenyl]-N-(5-isopropyl-1,3-thiazol-2-yl)propanamide;
37. N-1-(4-{2-[(5-isopropyl-1,3-thiazol-2-yl)amino]-2-oxoethyl}phenyl)glycinamide;
38. N-(4-{(1S)-2-[(5-isopropyl-1,3-thiazol-2-yl)amino]-1-methyl-2-oxoethyl}phenyl)nicotinamide;
39. N-(4-{(1R)-2-[(5-isopropyl-1,3-thiazol-2-yl)amino]-1-methyl-2-oxoethyl}phenyl)nicotinamide;
40. N-(4-{2-[(5-isopropyl-1,3-thiazol-2-yl)amino]-1-methyl-2-oxoethyl}phenyl)nicotinamide;
41. N-(4-{(1S)-2-[(5-isopropyl-1,3-thiazol-2-yl)amino]-1-methyl-2-oxoethyl}phenyl)pyridine-2-carboxamide;
42. N-(4-{(1R)-2-[(5-isopropyl-1,3-thiazol-2-yl)amino]-1-methyl-2-oxoethyl}phenyl)pyridine-2-carboxamide;
43. N-(4-{2-[(5-isopropyl-1,3-thiazol-2-yl)amino]-1-methyl-2-oxoethyl}phenyl)pyridine-2-carboxamide;
44. N-(4-{2-[(5-isopropyl-1,3-thiazol-2-yl)amino]-2-oxoethyl}phenyl)pyridine-2-carboxamide;
45. N-(4-{(1S)-2-[(5-isopropyl-1,3-thiazol-2-yl)amino]-1-methyl-2-oxoethyl}phenyl)isonicotinamide;
46. N-(4-{(1R)-2-[(5-isopropyl-1,3-thiazol-2-yl)amino]-1-methyl-2-oxoethyl}phenyl)isonicotinamide;
47. N-(4-{2-[(5-isopropyl-1,3-thiazol-2-yl)amino]-1-methyl-2-oxoethyl}phenyl)isonicotinamide;
48. N-(4-{2-[(5-isopropyl-1,3-thiazol-2-yl)amino]-2-oxoethyl}phenyl)isonicotinamide.

The compounds of formula (I), object of the invention, may be obtained by a process comprising reacting 2-amino-5-isopropyl-1,3-thiazole with a compound of formula (III)

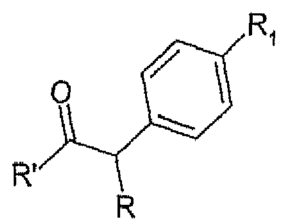

(III)

wherein R and $R_1$ are as above defined and R' is hydroxy or a suitable leaving group and, optionally, converting them into pharmaceutically acceptable salts thereof.
Alternatively, the compounds of formula (I) wherein $R_1$ is a group of formula (IIe), may be obtained by a process comprising: a') when $R_3$ is other than amino, reacting a compound of formula (IV)

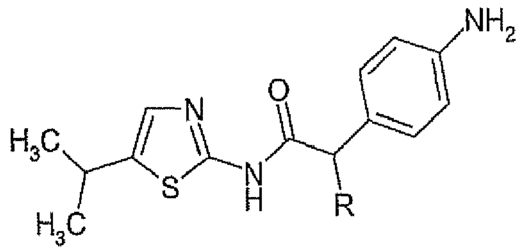

(IV)

with a compound of formula (V)

$R_3$-COX (V)

wherein R and $R_3$ are as above defined and X is hydroxy or a suitable leaving group;
a") when $R_3$ is amino, reacting a compound of formula (IV) with potassium cyanate; and, optionally, converting the compounds of formula (I) thus obtained in any one of steps a') or a") into pharmaceutically acceptable salts thereof.

The above process is an analogy process which can be carried out according to well known methods known in the art.

The reaction between 2-amino-5-isopropyl-1,3-thiazole with the compound of formula (III) wherein R' is hydroxy can be carried out in the presence of a coupling agent such as, for instance, a carbodiimide, i.e., 1,3-dicyclohexylcarbodiimide, 1,3-diisopropylcarbodiimide, 1-(3-dimethylaminopropyl)-3-ethylcarbodiimide, N-cyclohexylcarbodiimide, or N'-methyl-polystirene, optionally in the presence of a tertiary base such as triethylamine, N-methylmorpholine, N,N-diisopropylethylamine, pyridine or diethylaminomethyl-polystirene.

The reaction occurs in a suitable solvent such as, for instance, ethylacetate, dichloromethane, chloroform, tetrahydrofuran, diethyl ether, 1,4-dioxane, acetonitrile, toluene or N,N-dimethylformamide, at a temperature ranging from about -10°C to reflux, preferably from 0°C to room temperature, and for a suitable time, i.e., from about 30 minutes to about 8 days.

Alternatively, this same reaction may be also carried out according to a mixed anhydride method, that is by using an alkyl chloroformate such as ethyl, isobutyl or isopropyl chloroformate in the presence of a tertiary base such as triethylamine, N,N-diisopropylethylamine or pyridine.

Also this reaction is carried out in a suitable solvent such as, for instance, toluene, dichloromethane, chloroform, tetrahydrofuran, acetonitrile, diethyl ether, 1,4-dioxane or N,N-dimethylformamide, at a temperature ranging from about -30°C to room temperature.

The reaction between 2-amino-5-isopropyl-1,3-thiazole with the compound of formula (III) wherein R' is a suitable leaving group, for instance a halogen atom, can be carried out in the presence of a tertiary base such as triethylamine, N,N-diisopropylethylamine or pyridine, in a suitable solvent such as ethyl acetate, toluene, dichloromethane, chloroform, diethyl ether, tetrahydrofuran, acetonitrile or N,N-dimethylformamide, at a temperature ranging from about -10°C to reflux.

According to a preferred embodiment of the invention, the process for preparing the compounds of formula (I) is carried out by using the compounds of formula (III) wherein R' is hydroxy or a halogen atom, preferably chlorine.

It is clear to the skilled man that the amidation reaction according to step a') of the process is carried out in an analogous fashion, between the amino derivative of formula (IV) and the carboxylic acid derivative of formula (V), substantially as set forth above. As an example, the preparation of the compound of formula (I) wherein $R_3$ is methyl, according to step a'), is performed by reacting the derivative of formula (IV) with acetyl chloride (V) or, possibly, with analogous acylating agents thereof, e.g. acetic anhydride.

The reaction occurs in the presence of a suitable base such as triethylamine, N-methylmorpholine, N,N-diisopropylethylamine, pyridine or diethylamino-polystirene, in a suitable solvent such as ethylacetate, dichloromethane, tetrahydrofuran or acetonitrile, at a temperature ranging from 0°C to room temperature.

Within the compounds of formula (V), X is hydroxy or a suitable leaving group such as, for instance, a halogen atom. Preferably, X is hydroxy or a chlorine atom.

As per step a") of the process, the intermediate compound of formula (IV) is reacted with potassium cyanate, according to conventional methods for preparing ureido derivatives [compounds of formula (I) with $R_3$ as amino], in a suitable solvent, for instance acetonitrile, and in the presence of trifluoroacetic acid.

The optional salification of a compound of formula (I) or, alternatively, the conversion of a salt thereof into the free compound, can be all carried out according to conventional methods.

The starting 2-amino-5-isopropyl-1,3-thiazole is a known compound which can be easily obtained according to known methods, for instance as reported in the working examples.

Likewise, the compounds of formula (III) wherein R' is hydroxy or a suitable leaving group are known or may be prepared according to conventional methods.

As an example, the compounds of formula (III) wherein R' is hydroxy, R is as described above and $R_1$ is a group of formula (IIa) can be prepared by reacting the compounds of formula (VI) wherein R is as described above

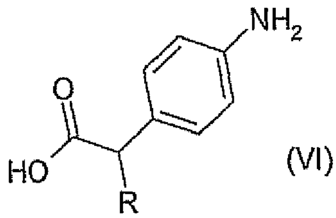

(VI)

with 6,6-dimethyl-5,7-dioxaspiro(2,5)octane-4,8-dione in a suitable solvent such as toluene, xylene or N,N-dimethylformamide, at refluxing temperature and for a time ranging from about 2 to about 6 hours. Alternatively, these same compounds can be prepared by reacting the above compounds of formula (VI) with γ-butyrolactone in a suitable solvent such as glacial acetic acid or hydrochloric acid, at a temperature ranging from 150 °C to 200 °C.
According to another alternative method, these compounds can be prepared by reacting the compounds of formula (VI) with 4-chlorobutyryl chloride in a suitable solvent such as chloroform, dichloromethane, N,N-dimethylacetamide at room temperature and, subsequently, by reaction with an inorganic base such as sodium or potassium hydrate at room temperature.

The compounds of formula (III) wherein $R_1$ is a group of formula (IIe) may be prepared by reacting the compounds of formula (VI) wherein R is as above defined, with a suitable carboxylic acid derivative of formula (V) so as to get any derivative of formula (III) wherein $R_3$ is other than amino or, alternatively, with potassium cyanate so as to get the derivative of formula (III) wherein $R_3$ is amino. The operative conditions therein employed are conventional and correspond to those previously reported, when referring to amidation reactions, or when referring to the preparation of ureido groups, respectively.

The compounds of formula (III) wherein R' is hydroxy, R is as described above and $R_1$ is a group (IIb) can be prepared by a process comprising:

a) reacting the compounds of formula (VI) with 2-chloroethylchloroformate in a suitable solvent such as chloroform or dichloromethane, at a temperature ranging from 0°C to room temperature and, subsequently, by treatment with sodium phosphate thus affording the compounds of formula (VII) wherein R is as described above

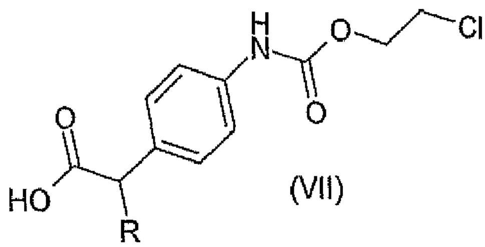

(VII)

b) and by cyclizing the compounds of formula (VII) in the presence of a suitable inorganic base such as potassium or sodium carbonate in N,N-dimethylformamide or in the presence of an organic base such as 1,5-diazabicyclo [4.3.0]non-5-ene or 1,8-diazabicyclo[5.4.0]undec-7-ene at room temperature.

The compounds of formula (III) wherein R' is hydroxy, R is as described above, $R_1$ is a group (IId) and $R_2$ is hydrogen can be prepared by a process comprising:

a) converting the compounds of formula (VI) wherein R is as described above, into the corresponding methyl ester derivatives (VIII) wherein R is as described above, by following conventional methods

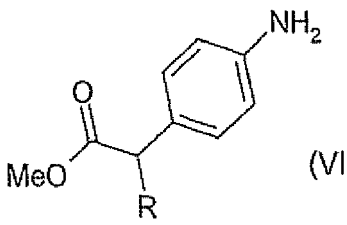

(VIII)

b) by reacting the compounds of formula (VIII) wherein R is as described above with chloroethylisocyanate in a suitable solvent such as chloroform, dichloromethane, tetrahydrofuran, 1,4-dioxane, at a temperature ranging from about 0°C to room temperature, thus affording the compounds of formula (IX) wherein R is as described above

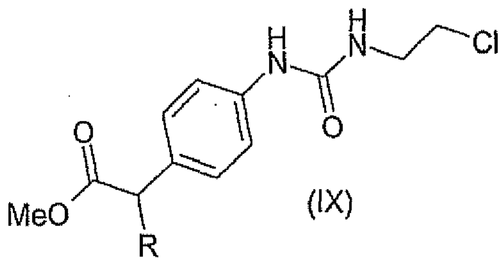

(IX)

c) by cyclizing the compounds of formula (IX) in the presence of a suitable inorganic base such as potassium or sodium carbonate in N,N-dimethylformamide or in the presence of an organic base such as 1,5-diazabicyclo[4.3.0]non-5-ene or 1,8-diazabicyclo[5.4.0]undec-7-ene at room temperature, thus affording the compounds of formula (X) wherein R is as described above

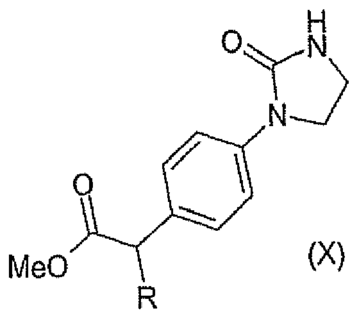

(X)

d) and by hydrolyzing the compounds of formula (X) with a base such as potassium or sodium carbonate or potassium or sodium or lithium hydrate, in a suitable solvent such as a water-methanol or water-tetrahydrofuran mixtures, at room temperature.

The compounds of formula (III) wherein R' is hydroxy, R is as described above, $R_1$ is a group of formula (IId) and $R_2$ is an alkyl group can be prepared by a process comprising:

a) reacting the compounds of formula (X) with the compounds of formula (XI) wherein $R_2$ is alkyl and X is halogen

$R_2$-X (XI)

in the presence of a base such as potassium tertbutoxide or sodium hydride, in a suitable solvent such as tetrahydrofuran, acetonitrile, 1,4-dioxane or N,N-dimethylformamide, at a temperature ranging from about 0°C to room temperature, thus affording the compounds of formula (XII) wherein R and $R_2$ are as described above

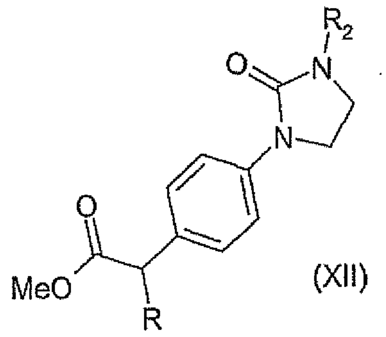

(XII)

b) hydrolyzing the compounds of formula (XII) with a base such as potassium or sodium carbonate or potassium or sodium or lithium hydrate in a suitable solvent such as a water-methanol or water-tetrahydrofuran mixture, at room temperature.

The compounds of formula (I) wherein R is as described above and $R_1$ is a group (IIc) can be prepared by reacting the compounds of formula (IV) wherein R is as described above with 2,2-dimethyl-4-oxo-1,3-dioxolane-5-acetaldehyde, for instance prepared as described in Tetrahedron Lett., 39, (1998), 5313-5316, in the presence of sodiumcyanoborohydride or (Polystyrylmethyl)trimethylammonium cyanoborohydride in a suitable solvent such as glacial acetic acid or trifluoroethanol, at room temperature.

The compounds of formula (IV) wherein R is as described above can be prepared by a process comprising:

a) reacting the compounds of formula (XIII) wherein R is as described above

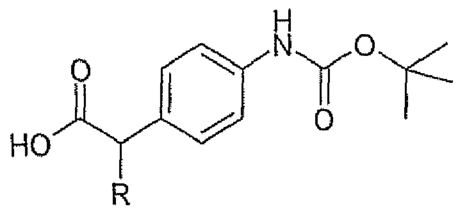

(XIII)

with 5-isopropyl-2-amino-1,3-thiazole, prepared by conventional methods, in the presence of a suitable coupling agent like a carbodiimide such as dicyclohexylcarbodiimide, diisopropylcarbodiimide, 1-(3-dimethylaminopropyl)-3-ethylcarbodiimide, affording the compounds of formula (XIV) wherein R is as described above

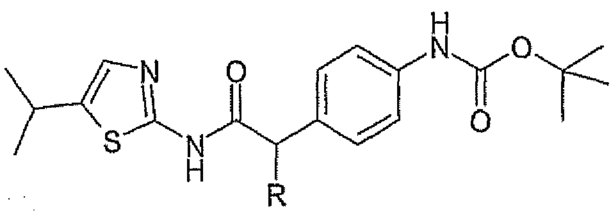

(XIV)

b) and hydrolyzing the compounds of formula (XIV) in acidic medium, for instance in the presence of hydrochloric acid or sulphuric acid in ethanol, or with formic acid or trifluoroacetic acid in dichloromethane, at room temperature for a time ranging from about 2 hours to about 12 hours.

The reaction according to the above step a) occurs in a suitable solvent such as, for instance, dichloromethane, chloroform, tetrahydrofuran, diethylether, 1,4-dioxane, acetonitrile, toluene or N,N-dimethylformamide at a temperature ranging from -10°C to room temperature. Alternatively, the compounds of formula (XIV) can be prepared by converting the compounds of formula (XIII) into the corresponding acyl chloride derivatives with thionyl chloride or oxalyl chloride in a suitable solvent such as toluene, dichloromethane, tetrahydrofuran, ethyl acetate, at a temperature ranging from about 0°C to room temperature and by subsequently reacting them with 5-isopropyl-2-amino-1,3-thiazole in the presence

of a suitable base such as triethylamine, N-methylmorpholine, N,N-diisopropylethylamine or pyridine, in a suitable solvent such as ethylacetate, dichloromethane, tetrahydrofuran, acetonitrile, N,N-dimethylformamide, at a temperature ranging from 0°C to room temperature.

The compounds of formula (XIII) wherein R is as described above can be prepared by reacting the compounds of formula (VI) with tertbutoxycarbonylanhydride in a suitable solvent such as mixtures water/1,4-dioxane, in the presence of a base such as sodium carbonate at room temperature for a time ranging from about 4 to about 12 hours.

The compounds of formula (III) wherein R' is a leaving group are conveniently prepared according to known methods by starting from the corresponding carboxylic acid derivatives of formula (III) wherein R' is hydroxy.
As an example, the compounds wherein R' is a halogen atom, for instance chlorine, are prepared by reacting the derivatives of formula (III) wherein R' is hydroxy with oxalyl or thionyl chloride, according to conventional methods for preparing acyl halides. This reaction is typically performed in the presence of a catalytic amounts of N,N-dimethylformamide and in the presence of a suitable solvent, for instance dichloromethane, tetrahydrofuran, ethyl acetate or toluene, at a temperature ranging from 0°C to reflux.

Also the compounds of formula (IV) may be prepared through amidation reactions as above reported, by reacting 2-amino-5-isopropyl-1,3-thiazole with a compound of formula (VIII).
In their turn, the compounds of formula (VIII) and (V), if not commercially available per se, are known or easily prepared according to known methods.

From all of the above, it is clear to the skilled man that when preparing the compounds of formula (I) according to any one of the aforementioned process variants, optional functional groups within the starting materials or the intermediates thereof and which could give rise to unwanted side reactions, need to be properly protected according to conventional techniques.
Likewise, the conversion of these latter into the free deprotected compounds may be carried out according to known procedures.
Just as an example, amino groups can be conventionally protected as (BOC) tert-butoxycarbonyl-amino groups through reaction with di-tert-butyl-dicarbonate, in a suitable solvent such as water/1,4-dioxane mixtures and in the presence of a base, e.g. sodium carbonate, by operating at room temperature and for a time varying from about 4 hours to about 12 hours.
Any subsequent deprotection may be thus performed by acidic hydrolysis, for instance in the presence of hydrochloric or sulphuric acid in ethanol, or with formic or trifluoroacetic acid in dichloromethane, by operating at room temperature for a time varying from about 2 hours to about 12 hours.

As it will be readily appreciated, if the compounds of formula (I) prepared according to the process described above are obtained as an admixture of isomers, their separation into the single isomers of formula (I), according to conventional techniques, is within the scope of the present invention.
Conventional techniques for racemate resolution include, for instance, partitioned crystallization of diastereoisomeric salt derivatives or preparative chiral HPLC.

## Pharmacology

The compounds of formula (I) are active as cdk/cyclin inhibitors and they may be used in the treatment of various tumors such as, for instance, carcinomas, e.g. mammary carcinoma, lung carcinoma, bladder carcinoma, colon carcinoma, ovary and endometrial tumors, sarcomas, e.g. soft tissue and bone sarcomas, and the hematological malignancies such as, e.g., leukemias.
In addition, the compounds of formula (I) are also useful in the treatment of other cell proliferative disorders such as psoriasis, vascular smooth cell proliferation associated with atherosclerosis and post-surgical stenosis and restenosis and in the treatment of Alzheimer's disease.
The inhibiting activity of putative cdk/cyclin inhibitors was determined first with a method based on the use of the MultiScreen-PH 96 well plate (Millipore), in which phosphocellulose filter paper was placed at each well bottom allowing binding of positive charged substrate after a washing/filtration step. When a radioactivity labelled phosphate moiety was transferred by the ser/threo kinase to the filter-bound histone, light emitted was measured in a scintillation counter, according to the following protocol:
Kinase reaction: 1.5 microM histone Hl substrate, 25 microM ATP (0.5 microCi $P^{33}$ gammaATP), 100 ng Cyclin A/cdk2 complex, 10 microM inhibitor in a final volume of 100 microl buffer (TRIS HCl 10 mM pH 7.5, $MgCl_2$ 10 mM, 7.5 mM DTT) were added to each well of a 96 U bottom well plate. After 10 minutes at 37°C incubation, reaction was stopped by 20 microl EDTA 120 mM.
Capture: 100 microl were transferred from each well MultiScreen plate, to allow substrate binding phosphocellulose filter. Plates were then washed 3 times with 150 microl/well PBS Ca++/Mg++ free and filtered by MultiScreen filtration system.
Detection: filters were allowed to dry at 37°C, then 100 microl/well scintillant were added and 33P labelled histone H1

was detected by radioactivity counting in the Top-Count instrument.

Results: data were analysed and expressed as % inhibition referred to total activity of enzyme (=100%).

When the number of compounds to be tested became consistent, the need for a fast and homogeneous screening assay was raised. Thus a method of assay based on the use of the SPA technology (Amersham Pharmacia Biotech) was set up. The assay consists of the transfer of radioactivity labelled phosphate moiety by the kinase to a biotinylated substrate. The resulting 33P-labolled biotinylated product is allowed to bind to streptavidin-coated SPA beads (biotin capacity 130pmol/mg), and light emitted was measured in a scintillation counter.

**Inhibition assay of cdk2/Cyclin A activity**

Kinase reaction: 4 microM in house biotinylated histone H1 (Sigma # H-5505) substrate, 10 microM ATP (0.1 microCi $P^{33}$gamma-ATP), 4.2 ng cdk2/Cyclin A complex, inhibitor in a final volume of 30 microl buffer (TRIS HCl 10 mM pH 7.5, $MgCl_2$ 10 mM, DTT 7.5 mM + 0.2 mg/ml BSA) were added to each well of a 96 U bottom. After 30 min at r.t. incubation, reaction was stopped by 100 microl PBS + 32 mM EDTA + 0.1% Triton X-100 + 500 microM ATP, containing 1 mg SPA beads. Then a volume of 110 microl is transferred to Optiplate.

After 20 min. incubation for substrate capture, 100 microl 5M CsCl were added to allow statification of beads to the top of the plate and let stand 4 hours before radioactivity counting in the Top-Count instrument

IC50 determination: inhibitors were tested at different concentrations ranging from 0.0015 to 10 microM. Experimental data were analyzed by the computer program GraphPad Prizm using the four parameter logistic equation:

$$y = bottom+(top-bottom)/(1+10^{\wedge}((logIC50-x)*slope))$$

where x is the logarithm of the inhibitor concentration, y is the response; y starts at bottom and goes to top with a sigmoid shape.

**Ki on cdk2/Cyclin A**

Experimental method: Reaction was carried out in buffer (10 mM Tris, pH 7.5, 10 mM $MgCl_2$, 0.2 mg/ml BSA, 7.5 mM DTT) containing 3.7 nM enzyme, histone and ATP (constant ratio of cold/labeled ATP 1/3000). Reaction was stopped with EDTA and the substrate captured on phosphomembrane (Multiscreen 96 well plates from Millipore). After extensive washing, the multiscreen plates are read on a top counter. Control (time zero) for each ATP and histone concentrations was measured.

Experimental design: Reaction velocities are measured at different four ATP, substrate (histone) and inhibitor concentrations. An 80-point concentration matrix was designed around the respective ATP and substrate Km values, and the inhibitor IC50 values (0.3, 1, 3, 9 fold the Km or IC50 values). A preliminary time course experiment in the absence of inhibitor and at the different ATP and substrate concentrations allow the selection of a single endpoint time (10 min) in the linear range of the reaction for the Ki determination experiment.

Kinetic parameter estimates: Kinetic parameters were estimated by simultaneous nonlinear least-square regression using [Eq.1] (competitive inhibitor respect to ATP, random mechanism) using the complete data set (80 points):

$$v = \frac{Vm \circ A \circ B}{\alpha \circ Ka \circ Kb + \alpha \circ Ka \circ B + a \circ Kb \circ A + A \circ B + \alpha \circ \frac{Ka}{Ki} \circ I \circ (Kb + \frac{B}{\beta})} \quad \text{[Eq.1]}$$

where A=[ATP], B=[Substrate], I=[inhibitor], Vm= maximum velocity, Ka, Kb, Ki the dissociation constants of ATP, substrate and inhibitor respectively. alpha and beta the cooperativity factor between substrate and ATP binding and substrate and inhibitor binding respectively.

In addition the selected compounds have been characterized on a panel of ser/threo kinases strictly related to cell cycle (cdk2/CyclinE, cdk1/CyclinB1, cdk5/p25, cdk4/CyclinD1).

**Inhibition assay of cdk2/CyclinE activity**

Kinase reaction: 10 microM in house biotinylated histone H1 (Sigma # H-5505) substrate, 30 microM ATP (0.3 microCi $P^{33}$gamma-ATP), 4 ng GST-Cyclin E/Cdk2 complex, inhibitor in a final volume of 30 microl buffer (TRIS HCl 10

mM pH 7.5, $MgCl_2$ 10 mM, DTT 7.5 mM + 0.2 mg/ml BSA) were added to each well of a 96 U bottom. After 60 min at r.t. incubation, reaction was stopped by 100 microl PBS + 32 mM EDTA + 0.1% Triton X-100 + 500 microM ATP, containing 1 mg SPA beads.
Then a volume of 110 microl is transferred to Optiplate.
After 20 min. incubation for substrate capture, 100 microl 5M CsCl were added to allow statification of beads to the top of the plate and let stand 4 hours before radioactivity counting in the Top-Count instrument
IC50 determination: see above

**Inhibition assay of cdk1/Cyclin B1 activity**

Kinase reaction: 4 microM in house biotinylated histone H1 (Sigma # H-5505) substrate, 20 microM ATP (0.2 microCi $P^{33}$gamma-ATP), 3 ng cdk1/CyclinB complex, inhibitor in a final volume of 30 microl buffer (TRIS HCl 10 mM pH 7.5, $MgCl_2$ 10 mM, DTT 7.5 mM + 0.2 mg/ml BSA) were added to each well of a 96 U bottom. After 20 min at r.t. incubation, reaction was stopped by 100 microl PBS + 32 mM EDTA + 0.1% Triton X-100 + 500 microM ATP, containing 1 mg SPA beads. Then a volume of 110 microl is transferred to Optiplate.
After 20 min. incubation for substrate capture, 100 microl 5M CsCl were added to allow statification of beads to the top of the Optiplate and let stand 4 hours before radioactivity counting in the Top-Count instrument.
IC50 determination: see above

**Inhibition assay of cdk5/p25 activity**

The inhibition assay of cdk5/p25 activity was performed according to the following protocol.
Kinase reaction: 10 microM biotinylated histone H1 (Sigma # H-5505) substrate, 30 microM ATP (0.3 microCi $P^{33}$ gamma-ATP), 15 ng CDK5/p25 complex, inhibitor in a final volume of 30 microl buffer (TRIS HCl 10 mM pH 7.5, $MgCl_2$ 10 mM, DTT 7.5 mM + 0.2 mg/ml BSA) were added to each well of a 96 U bottom. After 30 min at r.t. incubation, reaction was stopped by 100 microl PBS + 32 mM EDTA + 0.1.% Triton X-100 + 500 microM ATP, containing 1 mg SPA beads.
Then a volume of 110 microl is transferred to Optiplate.
After 20 min. incubation for substrate capture, 100 microl 5M CsCl were added to allow statification of beads to the top of the plate and let stand 4 hours before radioactivity counting in the Top-Count instrument.
IC50 determination: see above

**Inhibition assay of cdk4/Cyclin D1 activity**

Kinase reaction: 0,4 uM microM mouse GST-Rb (769-921) (# sc-4112 from Santa Cruz) substrate, 10 microM ATP (0.5 microCi $P^{33}$ gamma-ATP), 100 ng of baculovirus expressed GST-cdk4/GST-CyclinD1, suitable concentrations of inhibitor in a final volume of 50 microl buffer (TRIS HCl 10 mM pH 7.5, $MgCl_2$ 10 mM, 7.5 mM DTT+ 0.2 mg/ml BSA) were added to each well of a 96 U bottom well plate. After 40 min. at 37°C incubation, reaction was stopped by 20 microl EDTA 120 mM.

Capture: 60 microl were transferred from each well to MultiScreen plate, to allow substrate binding to phosphocellulose filter. Plates were then washed 3 times with 150 microl/well PBS $Ca^{++}$/$Mg^{++}$ free and filtered by MultiScreen filtration system.
Detection: filters were allowed to dry at 37°C, then 100 microl/well scintillant were added and $^{33}P$ labeled Rb fragment was detected by radioactivity counting in the Top-Count instrument.
IC50 determination: see above

Given the above inhibition assays, the compounds of formula (I) of the invention resulted to possess a remarkable cdk inhibitory activity.
As an example, when tested against cdk2/cyclin A, the representative compound of the invention (2S)-2-[4-(acetylamino) phenyl]-N-(5-isopropyl-1,3-thiazol-2-yl)propanamide showed an inhibitory activity, expressed as $IC_{50}$, of 11 nM.
Surprisingly, the said inhibitory activity resulted to be markedly superior than that of a very close prior art compound, used for comparative purposes. In fact, when tested against cdk2/cyclin A as formerly reported, the compound N-(5-isopropyl-1,3-thiazol-2-yl)-2-[4-(acetylamino)phenyl]acetamide of WO 01/14353 (see example 4, bridging paragraph on pages 32, 33 of the same), showed an $IC_{50}$ value of 110 nM.
Another representative compound of the invention, namely N-(5-isopropyl-1,3-thiazol-2-yl)-2-[4-(2-oxo-1-pirrolidinyl)phenyl]acetamide showed, when tested against cdc2/cyclin A, similar markedly superior activity.
So far, the novel compounds of the invention are unexpectedly endowed with a cdk inhibitory activity significantly higher than that of the closest prior art compounds of WO 01/14353 and are thus particularly advantageous, in therapy, against proliferative ' disorders associated with an altered cell cycle dependent kinase activity.
By restricting the unregulated proliferation of tumor cells, the compounds of formula (I) are therefore useful in therapy

in the treatment of various tumors such as, for instance, carcinomas, e.g., mammary carcinoma, bladder carcinoma, colon carcinoma, ovary endometrial tumors, sarcomas, e.g., soft tissue and bone sarcomas, and the hematological malignancies such as, e.g., leukemias.
In addition, the compounds of formula (I) are also useful in the treatment of other cell proliferative disorders such as psoriasis, vascular smooth cell proliferation associated with atherosclerosis and post-surgical stenosis and restenosis, and in the treatment of Alzheimer's disease.
The compounds of the present invention can be administered either as single agents or, alternatively, in combination with known anticancer treatments such as radiation therapy or chemotherapy regimen in combination with cytostatic or cytotoxic agents, antibiotic-type agents, alkylating agents, antimetabolite agents, hormonal agents, immunological agents, interferon-type agents, cyclooxygenase inhibitors (e.g. COX-2 inhibitors), matrixmetalloprotease inhibitors, telomerase inhibitors, tyrosine kinase inhibitors, anti-growth factor receptor agents, anti-HER agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors, and the like.
As an example, the compounds of the invention can be administered in combination with one or more chemotherapeutic agents such as, for instance, exemestane, formestane, anastrozole, letrozole, fadrozole, taxane and derivatives such as paclitaxel or docetaxel, encapsulated taxanes, CPT-11, camptothecin derivatives, anthracycline glycosides, e.g., doxorubicin, idarubicin, epirubicin, etoposide, navelbine, vinblastine, carboplatin, cisplatin, estramustine phosphate, celecoxib, tamoxifen, raloxifen, Sugen SU-5416, Sugen SU-6668, Herceptin, and the like, optionally within liposomal formulations thereof.
If formulated as a fixed dose, such combination products employ the compounds of this invention within the dosage range described below and the other pharmaceutically active agent within the approved dosage range.
Compounds of formula (I) may be used sequentially with known anticancer agents when a combination formulation is inappropriate.

The compounds of formula (I) of the present invention, suitable for administration to a mammal, e.g., to humans, can be administered by the usual routes and the dosage level depends upon the age, weight, conditions of the patient and administration route.

For example, a suitable dosage adopted for oral administration of a compound of formula (I) may range from about 10 to about 500 mg per dose, from 1 to 5 times daily. The compounds of the invention can be administered in a variety of dosage forms, e.g., orally, in the form tablets, capsules, sugar or film coated tablets, liquid solutions or suspensions; rectally in the form suppositories; parenterally, e.g., intramuscularly, or through intravenous and/or intrathecal and/or intraspinal injection or infusion.
The present invention also includes pharmaceutical compositions comprising a compound of formula (I) or a pharmaceutically acceptable salt thereof in association with a pharmaceutically acceptable excipient, which may be a carrier or a diluent.
The pharmaceutical compositions containing the compounds of the invention are usually prepared following conventional methods and are administered in a suitable pharmaceutical form.
For example, the solid oral forms may contain, together with the active compound, diluents, e.g., lactose, dextrose saccharose, sucrose, cellulose, corn starch or potato starch; lubricants, e.g., silica, talc, stearic acid, magnesium or calcium stearate, and/or polyethylene glycols; binding agents, e.g., starches, arabic gum, gelatine methylcellulose, carboxymethylcellulose or polyvinyl pyrrolidone; disintegrating agents, e.g., starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. These pharmaceutical preparations may be manufactured in known manner, for example, by means of mixing, granulating, tabletting, sugar-coating, or film-coating processes.
The liquid dispersions for oral administration may be, e.g., syrups, emulsions and suspensions.
As an example, the syrups may contain, as carrier, saccharose or saccharose with glycerine and/or mannitol and sorbitol.
The suspensions and the emulsions may contain, as examples of carriers, natural gum, agar, sodium alginate, pectin, methylcellulose, carboxymethylcellulose, or polyvinyl alcohol.

The suspension or solutions for intramuscular injections may contain, together with the active compound, a pharmaceutically acceptable carrier, e.g., sterile water, olive oil, ethyl oleate, glycols, e.g., propylene glycol and, if desired, a suitable amount of lidocaine hydrochloride.
The solutions for intravenous injections or infusions may contain, as a carrier, sterile water or preferably they may be in the form of sterile, aqueous, isotonic, saline solutions or they may contain propylene glycol as a carrier.
The suppositories may contain, together with the active compound, a pharmaceutically acceptable carrier, e.g., cocoa butter, polyethylene glycol, a polyoxyethylene sorbitan fatty acid ester surfactant or lecithin.

With the aim of better illustrating the present invention, without posing any limitation to it, the following examples are now given.

**Example 1**

**Preparation of 2-amino-5-isopropyl-1,3-thiazole**

3-Methylbutyraldehyde (2 ml; 18.6 mmol) was dissolved in 15 ml of 1,4-dioxane. 40.4 ml (18.6 mmol) of a solution 2% v/v of bromine in 1,4-dioxane was dropped therein at 0°C. The mixture was maintained at room temperature under stirring for 2 hours, then 2.83 g (37.2 mmol) of thiourea and 5 ml of ethanol were added.
After 6 hours at room temperature the solution was evaporated to dryness, the residue was dissolved in $CH_2Cl_2$ and the product extracted with 1M hydrochloric acid; the aqueous layer was made basic by using 30% ammonium hydrate and extracted again with $CH_2Cl_2$. The organic phase was dried over sodium sulphate and evaporated under vacuum. The residue was chromatographed on a silica gel column, eluting with cyclohexane-ethylacetate to give 1.1 g (42% yield) of the title compound.
$^1$H-NMR (DMSO-$d_6$) $\delta$ ppm: 6.6 (s, 2H, NH2); 6.58 (s, 1H, thiazole CH); 2.9 (m, 1H, CHMe2); 1.18 (s, 3H, MeCHMe); 1.17 (s, 3H, MeCHMe).

Example 2

**2-(4-aminophenyl)propanoic acid**

10 g (0.05 mol) of 2-(4-nitrophenyl)propanoic acid were dissolved in a mixture of 5 ml of water and 100 ml of methanol and 0.65 g of Pd/C 5% were added. The mixture was submitted to hydrogenation at 60 psi for 2 hours at room temperature. After the separation of the catalyst by filtration on celite, the methanol was evaporated under vacuum and the title compound was crystallised from water on cooling (7 g; 85 % yield).
ESI (+) MS: m/z 166 (100, MH+);
1H-NMR (400 MHz, DMSO-d6) ppm: 7.08-6.71 (2d, 4H, CH-phenyl, J=7.1 Hz), 3.76 (q, 1H, CH-Me, J=7.6 Hz), 1.53 (d, 3H, CH3, 7.6 Hz).

Example 3

**(2S)-2-(4-aminophenyl)propanoic acid and (2R)-2-(4-aminophenyl)propionic acid**

To 4.34 g of 2-(4-aminophenyl)propanoic acid, 26 ml of water and 3.94g of L-(+)-tartaric acid were added. The mixture was heated at 80°C under stirring until complete solution. Heating was then stopped and the solution made to spontaneously cool. After 24 hours, 3.8 g of levorotatory tartrate were filtered and dried. The solution containing the dextrorotatory tartrate was concentrated under vacuum at 40°C to evaporate about 10 ml of water. The solution was then cooled at 30°C and 1.047 g of sodium hydrate were added.
(+)-(2S)-2-(4-aminophenyl)propanoic acid was collected by filtration and dried to give 1.36 g ($\alpha_D^{20}$=+73.0°; C=0.1 % in methanol). Treating the levorotatory tartrate with sodium hydrate, (-)-(2R)-2-(4-aminophenyl)propanoic acid was obtained.

**Example 4**

**[4-(2-oxo-1-pyrrolidinyl)phenyl]acetic acid**

8.8 g (0.058 mol) of 4-aminophenylacetic acid were dissolved in 350 ml of N,N-dimethylformamide and 10 g (0.058 mol) of 6,6-dimethyl-5,7-dioxaspiro(2,5)octane-4,8-dione were added and the mixture stirred under reflux for 8 hours. The solvent was then evaporated under vacuum and the crude purified by chromatography on a silica gel column by using a mixture hexane-ethylacetate 7/3 as eluent, thus affording 6.53 g (51 % yield) of the title compound.
ESI (+) MS: m/z 220 (100, MH+);
1H-NMR (400 MHz, DMSO-d6) ppm: 7.19-7.68 (2d, 4H, CH-phenyl, J=8.7 Hz), 3.43 (s, 2H, CH2Ph), 3.85 (m, 2H, CH2N), 2.57 (m, 2H, CH2CO), 2.10 (m, 2H, CH2).
By working in an analogous way the following compounds can be prepared, by starting from the proper carboxylic acid derivatives:
2-[4-(2-oxo-1-pyrrolidinyl)phenyl]propanoic acid;
ESI (+) MS: m/z 234 (100, MH+);
1H-NMR (400 MHz, DMSO-d6) ppm: 7.17-7.66 (2d, 4H, CH-phenyl, J=8.6 Hz), 3.76 (q, 1H, CHMe, J=7.6 Hz), 1.53 (d, 3H, CH3, J=7.6 Hz), 3.84 (m, 2H, CH2N), 2.61 (m, 2H, CH2CO), 2.15 (m, 2H, CH2).
(2R)-2-[4-(2-oxo-1-pyrrolidinyl)phenyl]propanoic acid;

(2S)-[4-(2-oxo-1-pyrrolidinyl)phenyl]propanoic acid.

**Example 5**

**N-(5-isopropyl-1,3-thiazol-2-yl)-2-[4-(2-oxo-1-pyrrolidinyl)phenyl]acetamide**

2.5 g (11.4 mmol) of [4-(2-oxo-1-pyrrolidinyl)phenyl]acetic acid were dissolved in 150 ml of dichloromethane and 1.54 g (11.4 mmol) of N-hydroxybenzotriazole and 2.18 g (11.4 mmol) of 1-(3-dimethylaminopropyl)-3-ethylcarbodiimide were added successively, at 0°C. After 30 minutes at the same temperature, 1.35 g (5.7 mmol) of 5-isopropyl-2-amino-1,3-thiazole dissolved in 40 ml of dichloromethane were added dropwise. After 6 hours at room temperature the mixture was washed with a saturated sodium hydrogenocarbonate solution. The organic layer was separated, dried over sodium sulphate and concentrated under reduced pressure. Tre crude was purified by chromatography on a silica gel column (hexane-ethylacetate 7/3) to give 1.17 g (60% yield) of the title compound.
ESI (+) MS: m/z 344 (100, MH+);
1H-NMR (400 MHz, DMSO-d6) ppm: 7.48-7.51 (m, 4H, CH-phenyl), 3.68 (s, 2H, CH2Ph), 7.51 (s, 1H, CH-thiazole), 3.91 (m, 1H, CH-isopropyl), 1.39 (d, 6H, CH3, J=7.1 Hz) 3.83 (m, 2H, CH2N), 2.60 (m, 2H, CH2CO), 2.15 (m, 2H, CH2).
By working in an analogous way the following compounds can be prepared, by starting from the proper carboxylic acid derivatives:

N-(5-isopropyl-1,3-thiazol-2-yl)-2-[4-(2-oxo-1-pyrrolidinyl)phenyl]propanamide;
(2R)-N-(5-isopropyl-1,3-thiazol-2-yl)-2-[4-(2-oxo-1-pyrrolidinyl)phenyl]propanamide;
(2S)-N-(5-isopropyl-1,3-thiazol-2-yl)-2-[4-(2-oxo-1-pyrrolidinyl)phenyl]propanamide;
N-(5-isopropyl-1,3-thiazol-2-yl)-2-[4-(2-oxo-1-imidazolidinyl)phenyl]acetamide;
N-(5-isopropyl-1,3-thiazol-2-yl)-2-[4-(2-oxo-1-imidazolidinyl)phenyl]propanamide;
(2R)-N-(5-isopropyl-1,3-thiazol-2-yl)-2-[4-(2-oxo-1-imidazolidinyl)phenyl]propanamide;
(2S)-N-(5-isopropyl-1,3-thiazol-2-yl)-2-[4-(2-oxo-1-imidazolidinyl)phenyl]propanamide.

**Example 6**

**(2S)-2-{4-[(tert-butoxycarbonyl)amino]phenyl}propanoic acid**

12 g (73 mmol) of (2S)-2-(4-aminophenyl)propanoic acid suspended in 140 ml of 1,4-dioxane and 140 ml of water were treated with 7.6 g (73 mmol) of sodium carbonate dissolved in 70 ml of water. The resulting solution, cooled to 4°C, was treated with 17.2 g (79 mmol) of tertbutoxycarbonylanhydride and stirred overnight allowing the temperature to reach room temperature. The solvent was evaporated, the aqueous phase was washed with 150 ml of ethyl acetate, diluted with 200 ml of the same solvent, treated, while stirring, with 130 ml of 1M aqueous potassium hydrogenosulphate. The organic layer was separated and the aqueous phase extracted with more ethyl acetate. The combined organic extracts, washed with brine and dried over sodium sulphate, were evaporated and 18.18 g (94 % yield) of the title compound were obtained after trituration with hexane and filtration.
ESI (+) MS: m/z 266 (100, MH+);
1H-NMR (400 MHz, DMSO-d6) ppm: 7.47-7.52 (m, 4H, CH-phenyl), 3.76 (q, 1H, CHMe, J=7.6 Hz), 1.53 (d, 3H, CH3, J=7.6), 1 51 (s, 9H, tertbutyl).
By working in an analogous the following compounds can be prepared, by starting from the proper carboxylic acid derivatives:

(2R)-2-{4-[(tert-butoxycarbonyl)amino]phenyl}propanoic acid;
2-{4-[(tert-butoxycarbonyl)amino]phenyl}propanoic acid;
4-[(tert-butoxycarbonyl)amino]phenylacetic acid;
ESI (+) MS: m/z 252 (100, MH+);
1H-MMR (400 MHz, DMSO-d6) ppm: 7.45-7.51 (2d, 4H, CH-phenyl, J=8.8 Hz), 3.43 (s, 2H, CH2Ph), 1 51 (s, 9H, tertbutyl).

**Example 7**

**(2S)-2-(4-aminophenyl)-N-(5-isopropyl-1,3-thiazol-2-yl)propanamide**

265 mg (1 mmol) of (2S)-2-{4-[(tert-butoxycarbonyl)amino]phenyl}propanoic acid were treated at 4°C with 0.146 ml (2 mmol) of thionyl chloride. The reaction mixture was stirred for 2.5 hours and the reaction temperature was gradually

raised to room temperature. The mixture was evaporated to dryness, taken up with tetrahydrofuran and evaporated thoroughly. This crude product was employed in the following step without further purification. It was dissolved in 3 ml of dry tetrahydrofuran and added dropwise to a solution of 113 mg (0.8 mmol) of 5-isopropyl-2-amino-1,3-thiazole and 0.14 ml (1 mmol) of triethylamine in 5 ml of tetrahydrofuran at room temperature. After 3 hours the solvent was evaporated, the crude redissolved in 5 ml of dichloromethane and 1 ml of trifluoroacetic acid was added. The mixture was stirred at room temperature for 4 hours. Evaporation of the solvent left an oil which was treated with a saturated solution of sodium hydrogenocarbonate and extracted several times with dichloromethane. The combined organic extracts were dried over sodium sulphate and evaporated to afford 115 mg (50 % yield overall) of the title compound.

By working in an analogous way the following compounds can be prepared, by starting from the proper carboxylic acid derivatives:

(2R)-2-(4-aminophenyl)-N-(5-isopropyl-1,3-thiazol-2-yl)propanamide;
2-(4-aminophenyl)-N-(5-isopropyl-1,3-thiazol-2-yl)propanamide;
(4-aminophenyl)-N-(5-isopropyl-1,3-thiazol-2-yl)acetamide.

**Example 8**

**2-(4-{[(2-chloroethoxy)carbonyl]amino}phenyl)propanoic acid**

1 g (6 mmol) of 2-(4-aminophenyl) propanoic acid was dissolved in 30 ml of dichloromethane. The mixture was cooled to 0°C and 1.24 ml (12 mmol) of 2-chloroethylchloroformate were added. After 30 minutes 2.27 g (12 mmol) of sodium phosphate dodecahydrate were added portionwise and the reaction mixture maintained under stirring for 3 hours. Hydrochloric acid 0.5 N was then added until acid pH and the product extracted with dichloromethane. The organic layer was dried over sodium sulfate and evaporated to give an oil that afforded, after crystallization from diisopropylether, 1.3 g (93 % yield) of the title compound.
1H-NMR (400 MHz, DMSO-d6) ppm: 7.47-7.58 (2d, 4H, CH-phenyl, J=9.0 Hz), 3.75 (q, 1H, CH-Me, J=7.6 Hz), 4.31 (m, 2H, CH2Cl), 3.62 (m, 2H, CH2O), 1.53 (d, 3H, CH3, 7.6 Hz)

By working in an analogous was the following compounds can be prepared, by starting from the proper carboxylic acid derivatives:

(2R)-2-(4-{[(2-chloroethoxy)carbonyl]amino}phenyl)propanoic acid;
(2S)-2-(4-{[(2-chloroethoxy)carbonyl]amino}phenyl)propanoic acid;
(4- {[(2-chloroethoxy)carbonyl]amino}phenyl)acetic acid;
1H-NMR (400 MHz, DMSO-d6) ppm: 7.44-7.60 (2d, 4H, CH-phenyl, J=8.8 Hz), 3.43 (s, 2H, CH2Ph), 4.30 (m, 2H, CH2Cl), 3.63 (m, 2H, CH2O).

**Example 9**

**2-[4-(2-oxo-1,3-oxazolidin-3-yl)phenyl]propanoic acid**

500 mg (1.85 mmol) of 2-(4-{[(2-chloroethoxy)carbonyl]amino}phenyl)propanoic acid were dissolved in 10 ml of N,N-dimethylformamide and 0.55 ml (3.7 mmol) of 1,8-diazabicyclo[5.4.0]undec-7-ene were added at room temperature. After 2 hours under stirring the mixture was poured into 150 ml of water acidified with hydrochloric acid 1 N and extracted thoroughly with ethyl acetate. The organic extracts were dried over sodium sulfate and evaporated to dryness affording 350 mg (80 % yield) of the title compound, crystallized from a mixture ethyl acetate-diisopropylether.
ESI (+) MS: m/z 236 (100, MH+);
1H-NMR (400 MHz, DMSO-d6) ppm: 7.08-7.35 (2d, 4H, CH-phenyl, J=8.6 Hz), 3.76 (q, 1H, CH-Me, J=7.6 Hz), 3.80 (m, 2H, CH2N), 4.45 (m, 2H, CH2O), 1.53 (d, 3H, CH3, 7.6 Hz)

By workingin an analogous way the following compounds can be prepared, by starting from the proper chloro derivatives:

(2R)-2-[4-(2-oxo-1,3-oxazolidin-3-yl)phenyl]propanoic acid;
(2S)-2-[4-(2-oxo-1,3-oxazolidin-3-yl)phenyl]propanoic acid;
[4-(2-oxo-1,3-oxazolidin-3-yl)phenyl]acetic acid;
ESI (+) MS: m/z 222 (100, MH+);
1H-NMR (400 MHz, DMSO-d6) ppm: 7.10-7.38 (2d, 4H, CH-phenyl, J=8.7 Hz), 3.43 (s, 2H, CH2Ph), 3.79 (m, 2H, CH2N), 4.45 (m, 2H, CH2O).

### Example 10

**N-(4-isopropyl-1,3-thiazol-2-yl)-2-[4-(2-oxo-1,3-oxazolidin-3-yl)phenyl]propanamide**

460 mg (1.96 mmol) of 2-[4-(2-oxo-1,3-oxazolidin-3-yl)phenyl]propanoic acid were suspended in 15 ml of dichloromethane and 0.22 ml (2.5 mmol) of oxalyl chloride and one drop of N,N-dimethylformamide were added at 0°C. After 2 hours under stirring the solvent was evaporated, the crude redissolved with 15 ml of tetrahydrofuran and dropped into a solution of 250 mg (1.764 mmol) 5-isopropyl-2-amine-1,3-thiazole and 2.2 ml (11.76 mmol) of N,N-diisopropylethylamine in 11 ml of the same solvent at 0°C. The reaction temperature was gradually raised to room temperature and after 5 hours the solvent was evaporated under vacuum, the crude redissolved in dichloromethane and washed with water. The organic layer was finally dried over sodium sulphate, evaporated and the residue chromatographed on a silica gel column, giving 450 mg (71 % overall) of the title compound.
ESI (+) MS: m/z 360 (100, MH+);
1H-NMR (400 MHz, DMSO-d6) ppm: 6.88 (s, 4H, CH-phenyl), 3.85 (q, 1H, CHMe, J=6.8 Hz), 1.25 (d, 3H, CH3, J=6.8 Hz) 7.55 (s, 1H, CH-thiazole), 3.90 (m, 1H, CH-isopropyl), 1.38 (d, 6H, CH3-isopropyl, J=7.0 Hz), 3.80 (m, 2H, CH2N), 4.40 (m, 2H, CH2O).

By working in an analogous was the following compounds can be prepared, by starting from the proper carboxylic acid derivatives:

N-(5-isopropyl-1,3-thiazol-2-yl)-2-[4-(2-oxo-1,3-oxazolidin-3-yl)phenyl]acetamide; ESI (+) MS: m/z 346 (100, MH+); 1H-NMR (400 MHz, DMSO-d6) ppm: 7.58 (m, 4H, CH-phenyl), 3.66 (s, 2H, CH2), 7.51 (s, 1H, CH-thiazole), 3.91 (m, 1H, CH-isopropyl), 1.39 (d, 6H, CH3-isopropyl, J=7.1 Hz), 3.79 (m, 2H, CH2N), 4.45 (m, 2H, CH2O).
(2R)-N-(5-isopropyl-1,3-thiazol-2-yl)-2-[4-(2-oxo-1,3-oxazolidin-3-yl)phenyl]propanamide;
(2S)-N-(5-isopropyl-1,3-thiazol-2-yl)-2-[4-(2-oxo-1,3-oxazolidin-3-yl)phenyl]propanamide;
N-(5-isopropyl-1,3-thiazol-2-yl)-2-[4-(3-methyl-2-oxo-1-imidazolidinyl)phenyl]acetamide;
N-(5-isopropyl-1,3-thiazol-2-yl)-2-[4-(3-methyl-2-oxo-1-imidazolidinyl)phenyl]propanamde
(2R)-N-(5-isopropyl-1,3-thiazol-2-yl)-2-[4-(3-methyl-2-oxo-1-imidazolidinyl)phenyl]propanamide;
(2S)-N-(5-isopropyl-1,3-thiazol-2-yl)-2-[4-(3-methyl-2-oxo-1-imidazolidinyl)phenyl]propanamide.

### Example 11

**methyl 2-(4-aminophenyl)propanoate**

10 g (0.06 mol) of 4-aminophenylpropanoic acid were suspended in 100 ml of methanol. The reaction mixture was cooled at 0°C and 10 ml of 96 % sulphuric acid were added dropwise. The resulting solution was maintained at room temperature overnight and then poured into icy water, basified with 30 % ammonium hydrate and extracted several times with dichloromethane. The organic phase was dried over sodium sulphate to give 10 g (93 % yield) of the title compound as an oil.
ESI (+) MS: m/z 180 (100, MH+);
1H-NMR (400 MHz, DMSO-d6) ppm: 6.50-6.94 (2d, 4H, CH-phenyl, J=8.6 Hz), 3.70 (q, 1H, CHMe, J=7.2 Hz), 1.48 (d, 3H, CH3, J=7.2 Hz), 3.50 (s, 3H, CH3O).

By working in an analogous was the following compounds can be prepared, by starting from the proper carboxylic acid derivatives:

methyl (2R)-2-(4-aminophenyl)propanoate;
methyl (2S)-2-(4-aminophenyl)propanoate;
methyl 2-(4-aminophenyl)acetate;
ESI (+) MS: m/z 166 (100, MH+);
1H-NMR (400 MHz, DMSO-d6) ppm: 6.49-7.00 (2d, 4H, CH-phenyl, J=8.0 Hz), 3.55 (s, 2H, CH2Ph), 3.48 (s, 3H, CH3O)

### Example 12

**methyl 2-[4-({[(2-chloroethyl)amino]carbonyl}amino)phenyl]propanoate**

700 mg (3.9 mmol) of methyl 2-(4-aminophenyl)propanoate were dissolved in 10 ml of dichloromethane and 0.4 ml (4.68 mmol) of 2-chloroethylisocyanate were added at room temperature. After 2 hours the formed precipitate was collected by filtration and essicated under vacuum, giving 1 g (90 % yield) of the title compound.

1H-NMR (400 MHz, DMSO-d6) ppm: 7.28-7.53 (2d, 4H, CH-phenyl, J=9.0 Hz), 3.69 (q, 1H, CHMe, J=7.1 Hz), 1.44 (d, 3H, CH3, J=7.1 Hz), 3.48 (s, 3H, CH3O), 3.50 (m, 2H, CH2Cl), 3.31 (m, 2H, CH2N).

By working in an analogous was the following compounds can be prepared, by starting from the proper amino derivatives:

methyl (2R)-2-[4-({[(2-chloroethyl)amino]carbonyl}amino)phenyl]propanoate;
methyl (2S)-2-[4-({[(2-chloroemyl)amino]carbonyl}amino)phenyl]propanoate;
methyl 2-[4-({[(2-chloroethyl)amino]carbonyl}amino)phenyl]acetate.

Example 13

**methyl 2-[4-(2-oxo-1-imidazolidinyl)phenyl]propanoate**

950 mg (3.3 mmol) of methyl 2-[4-({[(2-chloroethyl)amino]carbonyl}amino)phenyl]propanoate were dissolved in 20 ml of N,N-dimethylformamide and 2 ml of 1,8-diazabicyclo[5.4.0]undec-7-ene were added. The reaction mixture was maintained at room temperature for 6 hours. 500 ml of a mixture 1/1 hexane/ethylacetate were added and the organic phase washed with hydrochloric acid 1N, brine, dried over sodium sulfate and evaporated to dryness, affording 760 mg (92 % yield) of the title compound.
ESI (+) MS: m/z 249 (100, MH+);
1H-NMR (400 MHz, DMSO-d6) ppm: 7.19-6.82 (2d, 4H, CH-phenyl, J=8.6 Hz), 3.69 (q, 1H, CHMe, J=7.2 Hz), 1.48 (d, 3H, CH3, J=7.2 Hz), 3.50 (s, 3H, CH3O), 3.85 (m, 2H, CH2N), 3.48 (m, 2H, CH2NH).

By working in an analogous was the following compounds can be prepared, by starting from the proper chloro derivatives:

methyl (2R)-2-[4-(2-oxo-1-imidazolidinyl)phenyl]propanoate;
methyl (2S)-2-[4-(2-oxo-1-imidazolidinyl)phenyl]propanoate;
methyl 2-[4-(2-oxo-1-imidazolidinyl)phenyl]acetate.

**Example 14**

**2-[4-(2-oxo-1-imidazolidinyl)phenyl]propanoic acid**

850 mg (3.42 mmol) of methyl 2-[4-(2-oxo-1-imidazolidinyl)phenyl]propanoate were dissolved in 30 ml of methanol and 10 ml of a saturated sodium carbonate solution were added. After a night at room temperature methanol was evaporated and the aqueous phase extracted with ethyl acetate, acidified with 1N hydrochloric acid and extracted again with dichloromethane. The organic layer was dried over sodium sulphate and evaporated to give 400 mg (50 % yield) of the title compound.
ESI (+) MS: m/z 235 (100, MH+);
1H-NMR (400 MHz, DMSO-d6) ppm: 7.03-7.33 (2d, 4H, CH-phenyl, J=8.6 Hz), 3.76 (q, 1H, CHMe, J=7.6 Hz), 1.53 (d, 3H, CH3, J=7.6 Hz), 3.84 (m, 2H, CH2N), 3.48 (m, 2H, CH2NH).

By working in an analogous was the following compounds can be prepared, by starting from the proper ester derivatives:

(2R)-2-[4-(2-oxo-1-imidazolidinyl)phenyl]propanoic acid;
(2S)-2-[4-(2-oxo-1-imidazolidinyl)phenyl]propanoic acid;
2-[4-(2-oxo-1-imidazolidinyl)phenyl]acetic acid;
(2R)-2-[4-(3-methyl-2-oxo-1-imidazolidinyl)phenyl]propanoic acid;
(2S)-2-[4-(3-methyl-2-oxo-1-imidazolidinyl)phenyl]propanoic acid;
2-[4-(3-methyl-2-oxo-1-imidazolidinyl)phenyl]propanoic acid;
ESI (+) MS: m/z 249 (100, MH+);
1H-NMR (400 MHz, DMSO-d6) ppm: 7.14-7.35 (2d, 4H, CH-phenyl, J=8.5 Hz), 3.76 (q, 1H, CHMe, J=7.6 Hz), 1.52 (d, 3H, CH3, J=7.6 Hz), 3.20 (m, 2H, CH2NMe), 3.70 (m, 2H, CH2NPh), 2.75 (s, 3H, CH3N);
2-[4-(3-methyl-2-oxo-1-imidazolidinyl)phenyl]acetic acid.

**Example 15**

**methyl 2-[4-(3-methyl-2-oxo-1-imidazolidinyl)phenyl]propanoate**

248 mg (1 mmol) of methyl 2-[4-(2-oxo-1-imidazolidinyl)phenyl]propanoate in N,N-dimethylformamide under argon atmosphere, were treated with 120 mg (3 mmol) of sodium hydride 60 %. After 20 minutes 0.068 ml (1.1 mmol) of methyl iodide were added. After 2 hours the reaction was poured into icy water, diluted with 10 ml of hexane-ethylacetatel/l and washed twice with 2N hydrochloric acid. The organic phase was dried over sodium sulphate, evaporated, purified by chromatography (hexane-ethylacetate 6/4) to afford 105 mg (40 % yield) of the title compound.
ESI (+) MS: m/z 263 (100, MH+);
1H-NMR (400 MHz, DMSO-d6) ppm: 6.93-7.19 (2d, 4H, CH-phenyl, J=8.4 Hz), 3.69 (q, 1H, CHMe, J=7.2 Hz), 1.48 (d, 3H, CH3, J=7.2 Hz), 3.35 (m, 2H, CH2NMe), 3.70 (m, 2H, CH2NPh), 2.74 (s, 3H, CH3N), 3.44 (s, 3H, CH3O).
By working in an analogous was the following compounds can be prepared, by starting from the proper ester derivatives:

methyl (2R)-2-[4-(3-methyl-2-oxo-1-imidazolidinyl)phenyl]propanoate;
methyl (2S)-2-[4-(3-methyl-2-oxo-1-imidazolidinyl)phenyl]propanoate;
methyl 2-[4-(3-methyl-2-oxo-1-imidazolidinyl)phenyl]acetate.

**Example 16**

**(2S)-2-{4-[(3S)-3-hydroxy-2-oxo-1-pyrrolidinyl]phenyl}-N-(5-isopropyl-1,3-thiazol-2-yl)propanamide**

158 mg (1 mmol) of [(4S)-2,2-dimethyl-5-oxo-1,3-dioxolan-4-yl]acetaldehyde and 290 mg (1 mmol) of (2S)-2-(4-aminophenyl)-N-(5-isopropyl-1,3-thiazol-2-yl)propanamide were dissolved in 10 ml of trifluoroethanol, treated with (Polystyrylmethyl)trimethylammonium cyanoborohydride (Novabiochem, loading: 3.0-4.5 mmol/g) and stirred overnight at room temperature. Under these conditions, the secondary amine derived from reductive amination spontaneously produced the $\gamma$-lactam. The resin was filtered off and washed with methanol. Evaporation of the filtrate followed by flash chromatography of the crude afforded 269 mg (72% yield) of the title compound.
By working in an analogous the following compounds can be prepared, by starting from the corresponding amino derivatives and the suitable (2,2-dimethyl-5-oxo-1,3-dioxolan-4-yl)acetaldehyde:

2-[4-(3-hydroxy-2-oxo-1-pyrrolidinyl)phenyl]-N-(5-isopropyl-1,3-thiazol-2-yl)acetamide;
2-{4-[(3S)-3-hydroxy-2-oxo-1-pyrrolidinyl]phenyl}-N-(5-isopropyl-1,3-thiazol-2-yl)acetamide;
2- {4-[(3R)-3-hydroxy-2-oxo-1-pyrrolidinyl]phenyl}-N-(5-isopropyl-1,3-thiazol-2-yl)acetamide;
2-[4-(3-hydroxy-2-oxo-1-pyrrolidinyl)phenyl]-N-(5-isopropyl-1,3-thiazol-2-yl)propanamide;
2-{4-[(3S)-3-hydroxy-2-oxo-1-pyrrolidinyl]phenyl}-N-(5-isopropyl-1,3-thiazol-2-yl)propanamide;
2-{4-[(3R)-hydroxy-2-oxo-1-pyrrolidinyl]phenyl}-N-(5-isopropyl-1,3-thiazol-2-yl)propanamide;
(2R)-2-{4-[(3S)-3-hydroxy-2-oxo-1-pyrrolidinyl]phenyl}-N-(5-isopropyl-1,3-thiazol-2-yl)propanamide;
(2S)-2-{4-[(3S)-3-hydroxy-2-oxo-1-pyrrolidinyl]phenyl}-N-(5-isopropyl-1,3-thiazol-2-yl)propanamide;
(2R)-2-{4-[(3R)-3-hydroxy-2-oxo-1-pyrrolidinyl]phenyl}-N-(5-isopropyl-1,3-thiazol-2-yl)propanamide;
(2S)-2-{4-[(3R)-3-hydroxy-2-oxo-1-pyrrolidinyl]phenyl}-N-(5-isopropyl-1,3-thiazol-2-yl)propanamide.

**Example 17**

**tert-Butyl 4-[(1S)-2-chloro-1-methyl-2-oxoethyl]phenylcarbamate**

(2S)-2-{4-[(tert-butoxycarbonyl)amino]phenyl}propanoic acid (4.2 g, 15.85 mmol) was dissolved in anhydrous tetrahydrofuran (48 mL), a drop of N,N-dimethylformamide was added and neat oxalyl chloride (1.49 mL, 17.43 mmol) was added dropwise at +4°C, under an argon atmosphere. The reaction mixture was stirred for 2 hours and the reaction temperature was gradually raised to room temperature. The mixture was evaporated to dryness; the off white solid was taken up with cyclohexane, filtered, washed thoroughly with cyclohexane and dried under vacum. The acyl chloride was obtained in 84% yield and employed without further purification in the following step.
By working in an analogous manner the following compounds can be prepared starting from the corresponding acid derivative:

tert-butyl 4-[(1R)-2-chloro-1-methyl-2-oxoethyl]phenylcarbamate;
tert-butyl 4-(2-chloro-1-methyl-2-oxoethyl)phenylcarbamate;

tert-butyl 4-(2-chloro-2-oxoethyl)phenylcarbamate.

**Example 18**

**tert-Butyl 4-{(1S)-2-[(5-isopropyl-1,3-thiazol-2-yl)amino]-1-methyl-2-oxoethyl}phenylcarbamate**

To 2-amino-5-isopropyl-1,3-thiazole (1.63 g, 11.48 mmol), dissolved in anhydrous tetrahydrofuran (50 mL), diethylaminomethyl-polystyrene (Fluka, loading: 3.2 $mmolg^{-1}$, 3.98 g) was added. The reaction mixture was gently stirred and treated drop-wise, at +4°C, under an argon atmosphere, with tert-butyl 4-[(1S)-2-chloro-1-methyl-2-oxoethyl]phenylcarbamate (3.61 g, 12.756 mmol) dissolved in tetrahydrofuran (40 mL). After 1 hour at +4°C followed by an additional 2 hours at room temperature, tris-(2-aminoethyl)-amine polystyrene (Novabiochem, loading: 3.2 $mmolg^{-1}$, 2.3 g) was added to sequester any unreacted acyl chloride together with its corresponding acid. After stirring for one hour the resins were filtered and washed several times with both dichloromethane and methanol. Evaporation of the volatiles left a light yellow amorphous solid that was subjected to the next step without any further purification.
ESI (+) MS: m/z 390 (100, MH+);
$^1$H NMR (400 MHz, DMSO-$d_6$): δ ppm: 11.98 (s, 1H), 9.24 (s, 1H), 7.35 (d, 2H, J = 8.7Hz), 7.2 (d, 2H, J = 8.7 Hz), 7.10 (s, 1H), 3.85 (q, 1H, J = 7.0 Hz), 3.1 (m, 1H, J = 6.8 Hz), 1.44 (s, 9H), 1.36, (d, 3H, J = 7.0 Hz), 1.22 (d, 6H, J = 6.8 Hz).
By working in an analogous manner the following compounds can be prepared starting from the corresponding acyl chloride derivative:

tert-butyl 4-{(1R)-2-[(5-isopropyl-1,3-thiazol-2-yl)amino]-1-methyl-2-oxoethyl}phenylcarbamate;
tert-butyl 4-{2-[(5-isopropyl-1,3-thiazol-2-yl)amino]-1-methyl-2-oxoethyl}phenylcarbamate;
tert-butyl 4-{2-[(5-isopropyl-1,3-thiazol-2-yl)amino]-2-oxoethyl}phenylcarbamate; m.p. 179-180°C;
$^1$H NMR (400 MHz, DMSO-$d_6$): δ ppm: 12.1 (s, 1H), 9.22 (s, 1H), 7.35 (d, 2H), 7.19 (s, 1H), 7.15 (d, 2H), 3.6 (s, 2H), 1.43 (s, 9H), 1.11 (d, 6H).

**Example 19**

**(2S)-2-(4-Aminophenyl)-N-(5-isopropyl-1,3-thiazol-2-yl)propanamide**

Crude tert-butyl 4- {(1S)-2-[(5-isopropyl-1,3-thiazol-2-yl)amino]-1-methyl-2-oxoethyl}phenylcarbamate was treated with an 8:2 mixture of dichloromethane and trifluoroacetic acid (130 mL), at room temperature with stirring. After 2.5 hours the volatiles were evaporated, the crude was dissolved in the minimum amount of methanol (~15 mL) and added dropwise to a saturated sodium bicarbonate aqueous solution (200 mL) with stirring. A white solid precipitated that was filtered and washed several times with water. After drying at 40°C, under vacuum, 2.96 g of an off white, crystalline solid (TLC: dichloromethane/methanol 95:5) were obtained in 89% yield (two steps).
ESI (+) MS: m/z 290 (100, MH+);
$^1$H NMR (400 MHz, DMSO-$d_6$): δ 11.86 (s, 1H), 7,10 (s, 1H), 6.97 (d, 2H, J = 2.0 Hz), 6.47 (d, 2H, J = 2.0 Hz), 4.92 (s, 2H), 3.85 (q, 1H, J = 7.0 Hz), 3.1 (m, 1H, J = 6.8 Hz), 1.31 (d, 3H, 7.0 Hz), 1.22 (d, 6H, J = 6.8 Hz);
Calcd for $C_{15}H_{19}N_3OS$: C, 62.25; H, 6.62; N, 14.52; S, 11.08. Found: C, 57.93; H, 6.16; N, 13.07; S, 9.53.
By working in an analogous manner the following compounds can be prepared starting from the corresponding tert-butyl carbamate:

(2R)-2-(4-aminophenyl)-N-(5-isopropyl-1,3-thiazol-2-yl)propanamide;
2-(4-aminophenyl)-N-(5-isopropyl-1,3-thiazol-2-yl)propanamide;
2-(4-aminophenyl)-N-(5-isopropyl-1,3-thiazol-2-yl)acetamide;
m.p. 165-166°C;
$^1$H NMR (400 MHz, DMSO-$d_6$): δ ppm: 11.98 (s, 1H), 7.13 (s, 1H), 7-6.6 (m, 4H), 5.9 (s, 2H), 3.55 (s, 2H), 3.08 (m, 1H), 1.12 (d, 6H).

**Example 20**

**(2S)-2-[4-(Acetylamino)phenyl]-N-(5-isopropyl-1,3-thiazol-2-yl)propanamide**

(2S)-2-(4-aminophenyl)-N-(5-isopropyl-1,3-thiazol-2-yl)propanamide (1.6 g, 5.54 mmol) was dissolved in pyridine (18 mL) and treated at +4°C, under an argon atmosphere, with neat acetic anhydride dropwise (627 μL, 6.648 mmol). After 1.5 hours the temperature was raised to room temperature and after another 1.5 hours the reaction mixture was added slowly to 300 mL of iced water, while stirring. Precipitation of a sticky solid occurred. Extraction of the aqueous

phase with dichloromethane (300 mL, 100 mL x 2) followed by washing of the organic phase first with 2 N HCl (200 mL), then with a saturated sodium bicarbonate aqueous solution (100 mL) and finally with a small amount of brine until neutral, drying over sodium sulphate and evaporation of the volatiles furnished 1.62 g of crude product. By further purification by column chromatography (dichloromethane, methanol 95:5) 1.58 g of pure compound were obtained as an off white amorphous solid (86% yield).
ESI (+) MS: m/z 332 (100, MH+);
$^1$H NMR (400 MHz, DMSO-$d_6$): δ 12.00 (s, 1H), 9.95 (s, 1H), 7.48 (d, 2H, J = 8.2 Hz), 7.24 (d, 2H, J = 8.2 Hz), 7.10 (s, 1H), 3.87 (q, 1H, 7.1 Hz), 3.05 (m, 1H, J = 6.8 Hz), 1.99(s, 3H), 1.37 (d, 3H, J = 7.1 Hz), 1.21 (d, 6H, J = 6.8 Hz);
Anal. Calcd for $C_{17}H_{21}N_3O_2S$: C, 61.61; H, 6.39; N, 12.68; S, 9.67. Found: C, 61.11; H, 6.45; N, 12.48; S, 9.31;
Enantiomeric excess: 99.5 % (chiral HPLC).

By working in an analogous manner the following compounds can be prepared starting from the corresponding amino derivative:

(2R)-2-[4-(acetylamino)phenyl]-N-(5-isopropyl-1,3-thiazol-2-yl)propanamide;
2-[4-(acetylamino)phenyl]-N-(5-isopropyl-1,3-thiazol-2-yl)propanamide.

**Example 21**

**<u>(2S)-2-{4-[(Aminocarbonyl)amino]phenyl}-N-(5-isopropyl-1,3-thiazol-2-yl)propanamide</u>**

(2S)-2-(4-aminophenyl)-N-(5-isopropyl-1,3-thiaxol-2-yl)propanamide (289 mg, 1 mmol) was dissolved in acetonitrile, treated with trifluoroacetic acid (153 μL, 2 mmol) and with potassium cyanate (162 mg, 2 mmol), at room temperature, stirred until homogeneous and left standing over night. The solvent was evaporated and the residue was taken up with ethyl acetate. The organic phase was washed first with a saturated sodium bicarbonate aqueous solution and then with brine, dried over sodium sulphate and evaporated. The resulting yellow oil was purified by column chromatography (dichloromethane, methanol 96:4) to afford 205 mg of the title compound as a white solid (yield: 62%).
ESI (+) MS: m/z 333 (100, MH+);
$^1$H NMR (400 MHz, DMSO-$d_6$): δ 11.95 (s, 1H), 8.6 (s, 1H), 7.30 (d, 2H, J = 8.7 Hz), 7.17 (d, 2H, J = 8.7 Hz), 7.10 (s, 1H), 5.75 (s, 2H), 3.83 (q, 1H, J = 6.9 Hz), 3.07 (m, 1H, J = 6.8 Hz), 1.36 (d, 3H, J = 6.9 Hz), 1.22 (d, 6H, J = 6.8 Hz);
Anal. Calcd for $C_{16}H_{20}N_4O_2S$: C, 57.81; H, 6.06; N, 16.85; S, 9.65. Found: C, 56.73; H, 6.06; N, 16.28; S, 7.54.

By working in an analogous manner the following compounds can be prepared starting from the corresponding amino derivative:

(2R)-2-{4-[(aminocarbonyl)amino]phenyl}-N-(5-isopropyl-1,3-thiazol-2-yl)propanamide;
2-{4-[(aminocarbonyl)amino]phenyl}-N-(5-isopropyl-1,3-thiazol-2-yl)propanamide;
2-{4-[(aminocarbonyl)amino]phenyl}-N-(5-isopropyl-1,3-thiazol-2-yl)acetamide.

**Example 22**

**<u>({4-[(S)-1-(5-Isopropyl-thiazol-2-ylcarbamoyl)-ethyl]-phenylcarbamoyl}-methyl)-carbamic acid tert-butyl ester</u>**

(2S)-2-(4-aminophenyl)-N-(5-isopropyl-1,3-thiazol-2-yl)propanamide (289 mg, 1 mmol) and Boc-gly-OH (210 mg, 1.2 mmol) dissolved in dichloromethane (10 mL) were treated with N-cyclohexylcarbodiimide, N'-methyl polystyrene (Novabiochem, loading: 1.93 $mmolg^{-1}$, 1.04 g). The mixture was gently stirred for one hour then kept standing at +4°C over night. The resin was filtered and washed thoroughly with dichloromethane. Evaporation of the solvent left a yellow oil (HPLC purity: 93%, measured at 254 nm), which was subjected to the next transformation without further purification.
ESI (+) MS: m/z 447 (100, MH+);
$^1$H NMR (400 MHz, DMSO-$d_6$): 12.00 (s, 1H), 9.95 (s, 1H), 7.49 (d, 2H, 8.5 Hz), 7.26 (d, 2H, J = 8.5), 7.11 (s, 1H), 6.96 (t, 1H, J = 6.1 Hz), 3.7 (q, 1H, J = 7.1 Hz), 3.26 (d, 2H, J = 6.1 Hz), 3.06 (m, 1H, J = 6.8 Hz), 1.35 (s, 9H), 1.37 (d, 3H, J = 7.1 Hz), δ 1.21 (d, 6H, J = 6.8 Hz).

By working in an analogous manner the following compounds can be prepared starting from the corresponding amino derivative:

({4-[(R)-1-(5-isopropyl-thiazol-2-ylcarbamoyl)-ethyl]-phenylcarbamoyl}-methyl)-carbamic acid tert-butyl ester;
({4-[1-(5-isopropyl-thiazol-2-ylcarbamoyl)-ethyl]-phenylcarbamoyl}-methyl)-carbamic acid tert-butyl ester;
({4-[(5-isopropyl-thiazol-2-ylcarbamoyl)-methyl]-phenylcarbamoyl}-methyl)-carbamic acid tert-butyl ester.

**Example 23**

**(2S)-2-[4-(Glycylamino)phenyl]-N-(5-isopropyl-1,3-thiazol-2-yl)propanamide**

({4-[(S)-1-(5-Isopropyl-thiazol-2-ylcarbamoyl)-ethyl]-phenylcarbamoyl}-methyl)-carbamic acid tert-butyl ester was treated with a 9:1 mixture of dichloromethane and trifluoroacetic acid (10mL) at room temperature, stirred for 1.5 hours then evaporated.
The remaining oil was dissolved in the minimum amount of absolute ethanol (~5. mL) and added drop-wise to a saturated sodium bicarbonate aqueous solution (50 mL), with stirring. The aqueous phase was extracted with dichloromethane, washed with brine, dried over sodium sulphate and evaporated. Purification of the crude by column chromatography (dichloromethane, methanol 9:1+1% TEA) afforded 217 mg of pure compound (overall yield: 63%).
ESI (+) MS: m/z 347 (100, MH+);
$^1$H NMR (400 MHz, DMSO-$d_6$): δ 7.54 (d, 2H, J = 8.7 Hz), 7.26 (d, 2H, J = 8.7 Hz), 7.11 (s, 1H), 1.21 (d, 6H, J = 6.8 Hz), 3.88 (q, 1H, J = 7.1 Hz), 3.05 (m, 1H, J = 6.8 Hz), 1.37 (d, 3H, J = 7.1 Hz);
Anal. Calcd for $C_{17}H_{22}N_4O_2S$: C, 58.94; H, 6.40; N, 16.17; S, 9.25. Found: C, 56.88; H, 6.56; N, 15.07; S, 7.69;
By working in an analogous manner the following compounds can be prepared starting from the corresponding carbamic acid tert-butyl ester:

(2R)-2-[4-(glycylamino)phenyl]-N-(5-isopropyl-1,3-thiazol-2-yl)propanamide;
2-[4-(glycylamino)phenyl]-N-(5-isopropyl-1,3-thiazol-2-yl)propanamide;
N-(4-{2-[(5-isopropyl-1,3-thiazol-2-yl)amino]-2-oxoethyl}phenyl)glycinamide.

**Example 24**

**N-(4-{(1S)-2-[(5-Isopropyl-1,3-thiazol-2-yl)amino]-1-methyl-2-oxoethyl}phenyl)nicotinamide**

Nicotinoyl chloride hydrochloride (762 mg, 4.15 mmol) in pyridine (30 mL), at +4°C, under an inert atmosphere, was treated, while stirring, with (2S)-2-(4-aminophenyl)-N-(5-isopropyl-1,3-thiazol-2-yl)propanamide (800 mg, 2.77 mmol) dissolved in pyridine (19 mL). After 2 hours the reaction was allowed to reach room temperature and left standing over night. The next day, the reaction was carried to completion by adding further nicotinoyl chloride hydrochloride (254 mg). The reaction mixture was added drop wise to crushed ice and water (600 mL), with stirring but the precipitate formed was rather sticky. The aqueous phase was extracted with ethyl acetate (300 mL, 100 mL x 2). The combined organic extracts were dried over sodium sulphate and the volatiles evaporated leaving 1.56 g of yellow oil. Further purification by chromatography (dichloromethane, methanol 95:5) afforded 967 mg of the title compound (yield: 89%).
ESI (+) MS: m/z 395 (100, MH+);
$^1$H NMR (400 MHz, DMSO-$d_6$): δ 12.05 (s, 1H), 10.40 (s, 1H), 9.04-9.09 (m, 1H), 8.70-8.76 (m, 1H), 8.22-8.28 (m, 1H), 7.69 (d, 2H, J = 8.5 Hz), 7.50-7.57 (m, 1H), 7.33 (d, 2H, J = 8.5 Hz), 7.12 (s, 1H), 3.92 (q, 1H, J = 7.1 Hz), 3.07 (m, 1H, J = 6.8 Hz), 1.40 (d, 3H, J = 7.1 Hz), 1.22 (d, 6H, J = 6.8 Hz);
Anal. Calcd for $C_{21}H_{22}N_4O_2S$: C, 63.94; H, 5.62; N, 14.20; S, 8.13. Found: C, 63.49; H, 5.65; N, 14.10; S, 8.10.
Enantiomeric excess: 99.5 % (chiral HPLC).
By working in an analogous manner the following compounds can be prepared starting from the corresponding amino derivative:

N-(4-{(1R)-2-[(5-isopropyl-1,3-thiazol-2-yl)amino]-1-methyl-2-oxoethyl}phenyl)nicotinamide;
N-(4-{2-[(5-isopropyl-1,3-thiazol-2-yl)amino]-1-methyl-2-oxoethyl}phenyl)nicotinamide.

**Example 25**

**N-(4-{(1S)-2-[(5-isopropyl-1,3-thiazol-2-yl)amino]-1-methyl-2-oxoethyl}phenyl)pyridine-2-carboxamide**

(2S)-2-(4-aminophenyl)-N-(5-isopropyl-1,3-thiazol-2-yl)propanamide (289 mg, 1 mmol) was dissolved in dichloromethane (10 ml) and treated first with picolinic acid (148 mg, 1.2 mmol) dissolved in the minimum amount of DMF then with N-cyclohexylcarbodiimide-N'-methyl polystyrene (Novabiochem, loading: 1.93 $mmolg^{-1}$, 1.04 g) and stirred on an orbital shaker over the week end. Since starting amino derivative was still present, more picolinic acid was added in portions (148 mg x 3) together with more N-cyclohexylcarbodiimide-N'-methyl polystyrene (1 g). The following day the solvents were evaporated and the crude was purified by chromatography (dichloromethane, ethyl acetate 8:2) furnishing 231 mg of pure compound as an of white amorphous solid (yield: 59%).
ESI (+) MS: m/z 395 (100, MH+);

$^1H$ NMR (400 MHz, DMSO-$d_6$): δ 12.04 (s, 1H), 10.57 (s, 1H), 8.70 (m, 1H), 8.12 (m, 1H), 8.04 (m, 1H), 7.80 (d, 2H, J = 8.5 Hz), 7.66 (m, 1H), 7.33 (d, 2H, J = 8.5 Hz), 7.11 (s, 1H), 3.91 (q, 1H, J = 7.1 Hz), 3.07 (m, 1H, J = 6.8 Hz), 1.41 (d, 3H, J = 7.1 Hz), 1.22 (d, 6H, J = 6.8 Hz);
Anal. Calcd for $C_{21}H_{22}N_4O_2S$: C, 63.94; H, 5.62; N, 14.20; S, 8.13. Found: C, 61.91; H, 5.71; N, 13.92; S, 7.31.

By working in an analogous manner the following compounds can be prepared starting from the corresponding amino derivative:

N-(4-{(1R)-2-[(5-isopropyl-1,3-thiazol-2-yl)amino]-1-methyl-2-oxoethyl}phenyl)pyridine-2-carboxamide;
N-(4-{2-[(5-isopropyl-1,3-thiazol-2-yl)amino]-1-methyl-2-oxoethyl}phenyl)pyridine-2-carboxamide;
N-(4- {2-[(5-isopropyl-1,3-thiazol-2-yl)amino]-2-oxoethyl}phenyl)pyridine-2-carboxamide.

**Example 26**

**N-(4-{(1S)-2-[(5-Isopropyl-1,3-thiazol-2-yl)amino]-1-methyl-2-oxoethyl}phenyl)isonicotinamide**

(2S)-2-(4-aminophenyl)-N-(5-isopropyl-1,3-thiazol-2-yl)propanamide (289 mg, 1 mmol) was dissolved in dichloromethane (10 ml) and treated first with isonicotinic acid (148 mg, 1.2 mmol) dissolved in the minimum amount of DMF then with N-cyclohexylcarbodiimide-N'-methyl polystyrene (Novabiochem, loading: 1.93 $mmolg^{-1}$, 1.04 g) and stirred on an orbital shaker over the week end. Since starting amino derivative (2S)-2-(4-aminophenyl)-N-(5-isopropyl-1,3-thiazol-2-yl)propanamide was still present, more isonicotinic acid was added in portions (148 mg x 3) together with more N-cyclohexylcarbodiimide-N'-methyl polystyrene (1 g, 0.5 g). After two days the solvents were evaporated and the crude was purified by chromatography (dichloromethane, methanol 9:1) furnishing 231 mg of pure compound as an off white amorphous solid (yield: 59%).
ESI (+) MS: m/z 395 (100, MH+);
$^1H$ NMR (400 MHz, DMSO-$d_6$): δ 12 04 (s, 1H), 10.45 (s, 1H), 8.75 (d, 2H, J = 6.1 Hz), 7.83 (d, 2H, J = 6.1 Hz), 7.68 (d, 2H, J = 8.5 Hz), 7.35 (d, 2H, J = 8.5), 7.12 (s, 1 H), 3.91 (q, 1H, J = 7.1 Hz), 3.07 (m, 1H, J = 6.8 Hz), 1.41 (d, 3H, J = 7.1 Hz), 1.23 (d, 6H, J = 6.8 Hz);
Anal. Calcd for $C_{21}H_{22}N_4O_2S$: C, 63.94; H, 5.62; N, 14.20; S, 8.13. Found: C, 62.74; H, 5.75; N, 13.94; S, 8.13.
Enantiomeric excess: 99.5 % (chiral HPLC).

By working in an analogous manner the following compounds can be prepared starting from the corresponding amino derivative:

N-(4-{(1R)-2-[(5-isopropyl-1,3-thiazol-2-yl)amino]-1-methyl-2-oxoethyl}phenyl)isonicotinamide;
N-(4-{2-[(5-isopropyl-1,3-thiazol-2-yl)amino]-1-methyl-2-oxoethyl}phenyl)isonicotinamide;
N-(4-{2-[(5-isopropyl-1,3-thiazol-2-yl)amino]-2-oxoethyl}phenyl)isonicotinamide.

**Example 27**

**2-[(4-{(1S)-2-[(5-Isopropyl-1,3-thiazol-2-yl)amino]-1-methyl-2-oxoethyl}phenyl)amino]-2-oxoethyl acetate**

(2S)-2-(4-aminophenyl)-N-(5-isopropyl-1,3-thiazol-2-yl)propanamide (3.6 g, 12.46 mmol) was dissolved in dichloromethane (60 mL), treated first with N,N-diisopropylethylamine (2.6 mL, 14.95 mmol) and then, at +4°C, under an argon atmosphere, with acetoxyacetyl chloride (1.61 mL, 14.95 mmol) in dichloromethane (40 mL). After one hour the reaction was diluted with dichloromethane, washed with 1 N HCl, then with a saturated sodium bicarbonate aqueous solution and finally with a small amount of brine until neutral. Drying over sodium sulphate and evaporation of the volatiles furnished in quantitative yield and in pure form the desired compound as a yellow amorphous solid.
ESI (+) MS: m/z 390 (100, MH+);
$^1H$ NMR (400 MHz, DMSO-$d_6$): δ 10.01 (s, 1H), 7.49 (d, 2H, J = 8.8 Hz), 7.28 (d, 2H, J = 8.8 Hz), 7.11 (s, 1H), 4.60 ((s, 2H), 3.88 (q, 1H, J = 6.9 Hz), 3.06 (m, 1H, J = 6.8 Hz), 2.09 (s, 3H), 1.38 (d, 3H, J = 6.9 Hz), 1.22 (d, 6H, J = 6.8 Hz);

By working in an analogous manner the following compounds can be prepared starting from the corresponding amino derivative:

2-[(4-{(1R)-2-[(5-isopropyl-1,3-thiazol-2-yl)amino]-1-methyl-2-oxoethyl}phenyl)amino]-2-oxoethyl acetate;
2-[(4-{2-[(5-isopropyl-1,3-thiazol-2-yl)amino]-1-methyl-2-oxoethyl}phenyl)amino]-2-oxoethyl acetate;
2-[(4-{2-[(5-isopropyl-1,3-thiazol-2-yl)amino]-2-oxoethyl}phenyl)amino]-2-oxoethyl acetate.

**Example 28**

**(2S)-2-[4-(Glycoloylamino)phenyl]-N-(5-isopropyl-1,3-thiazol-2-yl)propanamide**

2-[(4-{(1S)-2-[(5-Isopropyl-1,3-thiazol-2-yl)amino]-1-methyl-2-oxoethyl}phenyl)amino]-2-oxoethyl acetate (5 g, 12.85 mmol) in tetrahydrofuran (80 mL) was treated with lithium hydroxide monohydrate (1.08 g, 25.27 mmol) in water (80 mL) at room temperature. After one hour the reaction was partially evaporated, the aqueous phase was neutralized with 1M aqueous potassium hydrogenosulphate and extracted with ethylacetate. The organic phase was washed with brine, dried over sodium sulphate and evaporated. Purification of the crude by flash chromatography (dichloromethane/acetone 9:1) afforded 3.82 g of title compound as a white solid (yield: 86%).
ESI (+) MS: m/z 348 (100, MH+);
$^1$H NMR (400 MHz, DMSO-$d_6$): δ 12.01 (s, 1H), 9.58 (s, 1H), 7.60 (d, 2H, J = 8.5 Hz), 7.25 (d, 2H, J = 8.5 Hz), 7.11 (d, 1H, J = 1.0 Hz), 5.59 (t, 1H, J = 5.9 Hz), 3.93 (d, 2H, J = 5.9 Hz), 3.88 (q, 1H, J = 7.1 Hz), 3.05 (m, 1H, J = 6.9 Hz, J = 10 Hz), 1.38 (d, 3H, J = 7.1 Hz), 1.21 (d, 6H, J = 6.9 Hz);
Enantiomeric excess: 99.5 % (chiral HPLC);
$[\alpha]_D$ +207° (C=1, $CH_3OH$);
Anal. Calcd for $C_{17}H_{21}N_3O_3S$: C, 58.77; H, 6.09; N, 12.09; S, 9.23. Found: C, 58.58; H, 6.25; N, 11.65; S, 9.12.

By working in an analogous manner the following compounds can be prepared starting from the corresponding amino derivative:

(2R)-2-[4-(glycoloylamino)phenyl]-N-(5-isopropyl-1,3-thiazol-2-yl)propanamide;
2-[4-(glycoloylamino)phenyl]-N-(5-isopropyl-1,3-thiazol-2-yl)propanamide;
2-[4-(glycoloylamino)phenyl]-N-(5-isopropyl-1,3-thiazol-2-yl)acetamide.

## Claims

1. Use of a phenylacetamido-thiazole derivative represented by formula (I)

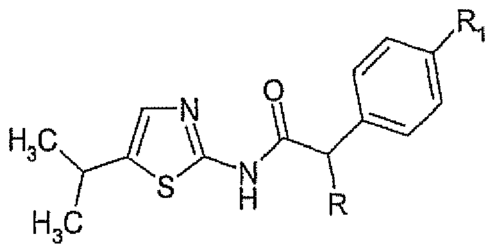

(I)

wherein
R is a hydrogen atom or a straight or branched $C_1$-$C_4$ alkyl group;
$R_1$ is a group of formula (IIa- d)

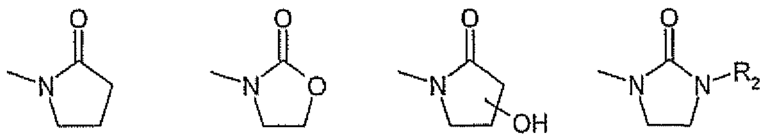

(IIa) (IIb) (IIc) (IId)

wherein
$R_2$ is hydrogen or a straight or branched $C_1$-$C_6$ alkyl group and the hydroxy group onto ring (IIc) is in any one of the free positions;
or a pharmaceutically acceptable salt thereof, for manufacturing a medicament for treating cell proliferative disorders associated with an altered cell cycle dependent kinase activity, by administration to a mammal in need thereof of an effective amount.

**2.** The use according to claim 1 wherein the cell proliferative disorder is selected from the group consisting of cancer, Alzheimer's disease, viral infections, auto-immune diseases and neurodegenerative disorders.

**3.** The use according to claim 2 wherein the cancer is selected from the group consisting of carcinoma, squamous cell carcinoma, hematopoietic tumors of myeloid or lymphoid lineage, tumors of mesenchymal origin, tumors of the central and peripheral nervous system, melanoma, seminoma, teratocarcinoma, osteosarcoma, xeroderma pigmentosum, keratocanthoma, thyroid follicular cancer, and Kaposi's sarcoma.

**4.** The use according to claim 1 wherein the cell proliferative disorder is selected from the group consisting of benign prostate hyperplasia, familial adenomatosis polyposis, neuro-fibromatosis, psoriasis, vascular smooth cell proliferation associated with atherosclerosis, pulmonary fibrosis, arthritis, glomerulonephritis and post-surgical stenosis and restenosis.

**5.** The use according to claim 1 wherein said treatment provides tumor angiogenesis and metastasis inhibition.

**6.** The use according to claims 1 wherein said medicament is for treatment or prevention of radiotherapy-induced or chemotherapy-induced alopecia.

**7.** The use according to claim 1 wherein said mammal in need thereof is subjected to a radiation therapy or chemotherapy regimen in combination with at least one cytostatic or cytotoxic agent.

**8.** The use according to claim 1 wherein the mammal in need thereof is a human.

**9.** Use of a compound as defined in claim 1 for manufacturing a medicament for inhibiting cyclin dependent kinase activity by contacting the said kinase with an effective amount.

**10.** A use according to claim 1 wherein said medicament comprises an effective amount of the compound of formula (1) N-(5-isopropyl-1,3-thiazol-2-yl)-2-[4-(2-oxo-1-pyrrolidinyl)phenyl]acetamide .

**11.** A phenylacetamido-thiazole derivative represented by formula (I)

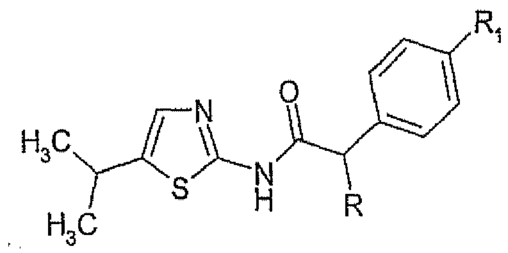

(I)

wherein
R is a hydrogen atom or a straight or branched $C_1$-$C_4$ alkyl group;
$R_1$ is a group of formula (IIa-d)

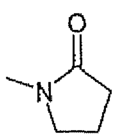

(IIa)

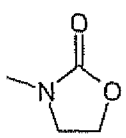

(IIb)

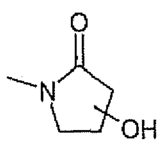

(IIc)

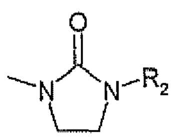

(IId)

wherein
$R_2$ is hydrogen or a straight or branched $C_1$-$C_6$ alkyl group and the hydroxy group onto ring (IIc) is in any one of the free positions;
or a pharmaceutically acceptable salt thereof.

**12.** A phenylacetamido-thiazole derivative of formula (I), according to claim 11, wherein R is methyl.

**13.** A phenylacetamido-thiazole derivative of formula (I), according to claim 11, wherein $R_1$ is a group of formula (IIa) or (IIb).

**14.** A phenylacetamido-thiazole derivative of formula (I), according to claim 11, wherein $R_1$ is a group of formula (IIc) and the hydroxy group thereof is in position 3 of the pyrrolidine ring.

**15.** A phenylacetamido-thiazole derivative of formula (I), according to claim 11, wherein R is a hydrogen atom.

**16.** The phenylacetamido-thiazole derivative of formula (I), according to claim 11, wherein R is a hydrogen atom and $R_1$ is a group of formula (IIa), which is N-(5-isopropyl-1,3-thiazol-2-yl)-2-[4-(2-oxo-1-pyrrolidinyl)phenyl]acetamide.

**17.** A phenylacetamido-thiazole derivative of formula (I), as defined in claim 11, optionally in the form of a pharmaceutically acceptable salt, selected from the group consisting of:

1) N-(5-isopropyl-1,3-thiazol-2-yl)-2-[4-(2-oxo-1-pyrrolidinyl)phenyl]acetamide;
2) N-(5-isopropyl-1,3-thiazol-2-yl)-2-[4-(2-oxo-1-pyrrolidinyl)phenyl]propanamide;
3) (2R)-N-(5-isopropyl-1,3-thiazol-2-yl)-2-[4-(2-oxo-1-pyrrolidinyl)phenyl]propanamide;
4) (2S)-N-(5-isopropyl-1,3-thiazol-2-yl)-2-[4-(2-oxo-1-pyrrolidinyl)phenyl]propanamide;
5) 2-[4-(3-hydroxy-2-oxo-1-pyrrolidinyl)phenyl]-N-(5-isopropyl-1,3-thiazol-2-yl)acetamide;
6) 2-{4-[(3S)-3-hydroxy-2-oxo-1-pyrrolidinyl]phenyl}-N-(5-isopropyl-1,3-thiazol-2-yl)acetamide;
7) 2-{4-[(3R)-3-hydroxy-2-oxo-1-pyrrolidinyl]phenyl}-N-(5-isopropyl-1,3-thiazol-2-yl)acetamide;
8) 2-[4-(3-hydroxy-2-oxo-1-pyrrolidinyl)phenyl]-N-(5-isopropyl-1,3-thiazol-2-yl)propanamide;
9) 2-{4-[(3S)-3-hydroxy-2-oxo-1-pyrrolidinyl]phenyl}-N-(5-isopropyl-1,3-thiazol-2-yl)propanamide;
10) 2-{4-[(3R)-hydroxy-2-oxo-1-pyrrolidinyl]phenyl}-N-(5-isopropyl-1,3-thiazol-2-yl)propanamide;
11) (2R)-2-{4-[(3S)-3-hydroxy-2-oxo-1-pyrrolidinyl]phenyl}-N-(5-isopropyl-1,3-thiazol-2-yl)propanamide;
12) (2S)-2-{4-[(3S)-3-hydroxy-2-oxo-1-pyrrolidinyl]phenyl}-N-(5-isopropyl-1,3-thiazol-2-yl)propanamide;
13) (2R)-2-{4-[(3R)-3-hydroxy-2-oxo-1-pyrrolidinyl]phenyl}-N-(5-isopropyl-1,3-thiazol-2-yl)propanamide;
14) (2S)-2-{4-[(3R)-3-hydroxy-2-oxo-1-pyrrolidinyl]phenyl}-N-(5-isopropyl-1,3-thiazol-2-yl)propanamide;
15) N-(5-isopropyl-1,3-thiazol-2-yl)-2-[4-(2-oxo-1,3-oxazolidin-3-yl)phenyl]acetamide;
16)N-(5-isopropyl-1,3-thiazol-2-yl)-2-[4-(2-oxo-1,3-oxazolidin-3-yl)phenyl]propanamide;
17) (2R)-N-(5-isopropyl-1,3-thiazol-2-yl)-2-[4-(2-oxo-1,3-oxazolidin-3-yl)phenyl]propanamide;
18) (2S)-N-(5-isopropyl-1,3-thiazol-2-yl)-2-[4-(2-oxo-1,3-oxazolidin-3-yl)phenyl]propanamide;
19) N-(5-isopiopyl-1,3-thiazol-2-yl)-2-[4-(2-oxo-1-imidazolidinyl)phenyl]acetamide;
20) N-(5-isopropyl-1,3-thiazol-2-yl)-2-[4-(2-oxo-1-inudazolidmyl)phenyl]propanamide;
21) (2R)-N-(5-isopropyl-1,3-thiazol-2-yl)-2-[4-(2-oxo-1-imidazolidinyl)phenyl]propanamide;
22) (2S)-N-(5-isopropyl-1,3-thiazol-2-yl)-2-[4-(2-oxo-1-imidazolidinyl)phenyl]propanamide;
23) N-(5-isopropyl-1,3-thiazol-2-yl)-2-[4-(3-methyl-2-oxo-1-imidazolidinyl)phenyl]acetamide;
24) N-(5-isopropyl-1,3-thiazol-2-yl)-2-[4-(3-methyl-2-oxo-1-imidazolidinyl)phenyl]propanamide;
25) (2R)-N-(5-isopropyl-1,3-thiazol-2-yl)-2-[4-(3-methyl-2-oxo-1-imidazolidinyl)phenyl]propanamide;
26) (2S)-N-(5-isopropyl-1,3-thiazol-2-yl)-2-[4-(3-methyl-2-oxo-1-imidazolidinyl)phenyl]propanamide;

**18.** A process for preparing the compounds of formula (I) or the pharmaceutically acceptable salts thereof, as defined in claim 11, which process comprises reacting 2-amino-5-isopropyl-1,3-thiazole with a compound of formula (III)

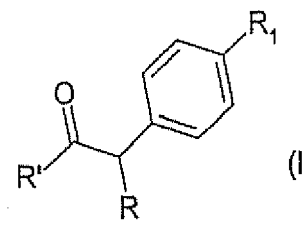

(III)

wherein R and $R_1$ are as defined in claim 11 and R' is hydroxy or a suitable leaving group and, optionally, converting them into pharmaceutically acceptable salts thereof.

**19.** A process according to claim 18 wherein R' is hydroxy or a halogen atom.

**20.** A process according to claim 19 wherein R' is hydroxy or a chlorine atom.

**21.** A pharmaceutical composition comprising a therapeutically effective amount of a phenylacetamido-thiazole derivative of formula (I), as defined in claim 11, and at least one pharmaceutically acceptable excipient, carrier and/or diluent.

**22.** A pharmaceutical composition according to claim 21 further comprising one or more chemotherapeutic agents.

**23.** A product or kit comprising a compound of formula (I) as defined in claim 11 or a pharmaceutical composition thereof as defined in claim 21, and one or more chemotherapeutic agents, as a combined preparation for simultaneous, separate or sequential use in anticancer therapy.

**24.** A compound of formula (I), as defined in claim 11, for us as a medicament.

**25.** Use of a compound of formula (I), as defined in claim 11, in the manufacture of a medicament with cell cycle dependent kinase activity.

**26.** Use of a compound of formula (I), as defined in claim 11, in the manufacture of a medicament with antitumor activity.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 261503 A
- WO 9804536 A
- EP 412404 A
- WO 9921845 A
- WO 9965884 A
- WO 0185707 A
- WO 0026202 A
- WO 0114353 A
- US 6114365 A


### Non-patent literature cited in the description


- **KEVIN R. WEBSTER et al.** *Exp. Opin. Invest. Drugs,* 1998, vol. 7 (6), 865-887
- *J. Biochem.,* 1995, vol. 117, 741-749
- *Tetrahedron Lett.,* 1998, vol. 39, 5313-5316